# EUROPÄISCHE PATENTSCHRIFT

(11) **EP 3 230 406 B1**
(45) Veröffentlichungstag und Bekanntmachung des Hinweises auf die Patenterteilung: **10.10.2018**
(21) Anmeldenummer: 15794080.0
(22) Anmeldetag: 11.11.2015
(51) Int. Cl.: C09B 57/00, C09K 19/34, C09K 19/60

(54) **VORRICHTUNG ZUR REGULIERUNG DES ENERGIE-DURCHTRITTS**
DEVICE FOR CONTROLLING THE PASSAGE OF ENERGY
DISPOSITIF POUR RÉGULER LE PASSAGE D'ÉNERGIE

(30) Priorität: 09.12.2014 EP 14004145
(43) Veröffentlichungstag der Anmeldung: 18.10.2017
(73) Patentinhaber: Merck Patent GmbH, 64293 Darmstadt (DE)
(72) Erfinder: KIRSCH, Peer, 64342 Seeheim-Jugenheim (DE); BECK, Susann, 64283 Darmstadt (DE); JUNGE, Michael, 64319 Pfungstadt (DE); BEYER, Andreas, 63452 Hanau (DE); PATWAL, Ursula, 64354 Reinheim (DE)
(86) Internationale Anmeldenummer: PCT/EP2015/002261
(87) Internationale Veröffentlichungsnummer: WO 2016/091345

(56) Entgegenhaltungen:
- WO-A1-2014/132978
- WO-A1-2014/180525
- WO-A1-2014/187529
- WO-A2-2013/005177

## Beschreibung

Die vorliegende Anmeldung betrifft Verbindungen und Vorrichtungen zur Regulierung des Energie-Durchtritts von einem Außenraum in einen Innenraum, spezielle Vorrichtungen wie Fenster und Verwendungen der Verbindungen und Vorrichtungen.

Unter einer Vorrichtung zur Regulierung des Energie-Durchtritts wird vorliegend allgemein eine Vorrichtung verstanden, welche den Durchtritt von Energie durch eine Fläche reguliert, welche relativ gesehen hoch energiedurchlässig ist. Bevorzugt ist diese relativ gesehen hoch energiedurchlässige Fläche innerhalb einer relativ gesehen weniger energiedurchlässigen Struktur angeordnet. Beispielsweise kann die hoch energiedurchlässige Fläche eine Glasfläche oder eine offene Fläche sein, und die weniger energiedurchlässige Struktur, welche die hoch energiedurchlässige Fläche enthält, kann eine Wand sein.

Bevorzugt wird durch die Vorrichtung der Durchtritt von Energie aus Sonneneinstrahlung, entweder direkt oder indirekt, reguliert.

Der regulierte Energiedurchtritt erfolgt von einem Außenraum, bevorzugt der unmittelbar der Sonneneinstrahlung ausgesetzten Umwelt, in einen Innenraum, beispielsweise ein Gebäude oder ein Fahrzeug, oder eine andere von der Umgebung weitgehend abgeschlossene Einheit.

Unter dem Begriff Energie wird im Rahmen der vorliegenden Erfindung insbesondere Energie durch elektromagnetische Strahlung im UV-A-, VIS-, sowie NIR-Bereich verstanden. Insbesondere wird darunter Energie durch Strahlung verstanden, welche von den üblicherweise in Fenstern verwendeten Materialien (z. B. Glas) nicht oder nur in einem vernachlässigbaren Umfang absorbiert wird. Gemäß den üblicherweise verwendeten Definitionen wird unter dem UV-A-Bereich eine Wellenlänge von 320 bis 380 nm verstanden, unter dem VIS-Bereich eine Wellenlänge von 380 nm bis 780 nm verstanden und unter dem NIR-Bereich eine Wellenlänge von 780 nm bis 2000 nm verstanden. Entsprechend wird unter dem Begriff Licht allgemein elektromagnetische Strahlung mit Wellenlängen zwischen 320 und 2000 nm verstanden.

Auf dem Gebiet der Vorrichtungen zur Regulierung des Energie-Durchtritts von einem Außenraum in einen Innenraum sind in den vergangenen Jahren mehrere unterschiedliche technische Lösungen vorgeschlagen worden.

Eine vorteilhafte Lösung ist die Verwendung von Schaltschichten enthaltend ein flüssigkristallines Medium in Kombination mit einem oder mehreren dichroitischen Farbstoffen. Durch Anlegen einer Spannung kann bei diesen Schaltschichten eine Veränderung der räumlichen Ausrichtung der Moleküle der dichroitischen Verbindung erzielt werden, welche eine Änderung ihrer Absorption und damit der Transmission durch die Schaltschicht bewirkt. Eine entsprechende Vorrichtung ist beispielsweise in WO 2009/141295 beschrieben.

Unter einem dichroitischen Farbstoff wird im Rahmen der vorliegenden Anmeldung eine lichtabsorbierende Verbindung verstanden, bei der die Absorptionseigenschaften von der Ausrichtung der Verbindung zur Polarisationsrichtung des Lichts abhängig sind. Typischerweise weist eine dichroitische Farbstoffverbindung eine langgestreckte Form auf, d.h. die Verbindung ist in einer Raumrichtung deutlich länger (Längsachse) als in den anderen beiden Raumrichtungen.

Alternativ kann eine solche Transmissionsänderung auch ohne elektrische Spannung durch einen Temperatur-induzierten Übergang von einem isotropen Zustand des flüssigkristallinen Mediums zu einem flüssigkristallinen Zustand erzielt werden, wie beispielsweise in US 2010/0259698 beschrieben.

Weiterhin ist es bekannt, Vorrichtungen mit einer Schaltschicht, enthaltend ein flüssigkristallines Medium mit mindestens einem dichroitischen Farbstoff, so zu gestalten, dass die von dem Farbstoff absorbierte Energie teilweise als Fluoreszenzstrahlung wieder emittiert wird, die ihrerseits auf eine Solarzelle geleitet wird, welche sie in elektrische Energie umwandelt (WO 2009/141295).

Es ist besonders von Interesse, zu den genannten Zwecken fluoreszierende Farbstoffe einzusetzen, die für ihre hohe Lichtechtheit bekannt sind. Dazu zählen Perylen- oder Terrylenderivate, deren Stabilität allerdings bei extremen Anforderungen für die Anwendung in Fenstern verbesserungsbedürftig ist. Benzothiadiazol- und Diketopyrrolopyrrolderivate erfüllen zwar die Anforderungen im Hinblick auf Lichtechtheit und Dichroismus, absorbieren allerdings oftmals zu kurzwellig, so dass sich der blau-grüne Farbbereich nur unzureichend abdecken lässt (Zhang et al., 2004, 14; WO2004/090046).

Li et al, 2014, beschreiben Derivate von Difluorbenzothiadiazolen und Verwendungen in organischen Photovoltaikzellen.

Bei den bekannten Vorrichtungen zur Regulierung des Energie-Durchtritts besteht hohes Interesse an der Entwicklung verbesserter Vorrichtungen und dazu geeigneter Verbindungen.

Der Erfindung liegt daher die Aufgabe zugrunde, neue, verbesserte Verbindungen und Vorrichtungen zur Regulierung des Energie-Durchtritts von einem Außenraum in einen Innenraum bereitzustellen, welche die oben beschriebenen Nachteile überwinden. Die Verbindungen sollen dabei insbesondere in einer Schaltschicht einsetzbar sein.

Der Erfindung liegt dabei insbesondere die Aufgabe zugrunde, neue Farbstoffe mit starker Fluoreszenz, hoher Lichtechtheit, einem hohen dichroitischen Verhältnis und guter Löslichkeit in typischen Flüssigkristallmischungen bereitzustellen. Die Verbindungen sollen dabei die besonderen Anforderungen erfüllen, die sich bei Anwendungen im Zusammenhang mit Fenstern stellen, insbesondere in aktiven, flüssigkristall-basieren Abschattungsvorrichtungen.

Die Verbindungen sollen eine starke Lichtabsorption im VIS- und/oder NIR-Bereich des Lichts aufweisen. Der Erfindung liegt insbesondere die Aufgabe zugrunde, Verbindungen bereitzustellen, die eine gute Absorption oberhalb 450 nm zeigen. Insbesondere sollen sie eine geringe Bandlücke und eine Absorptionsbande >450 nm aufweisen. Für Verbindungen und Vorrichtungen, die emittiertes Fluoreszenzlicht in elektrische Energie umwandeln, ist es weiterhin von Interesse, dass die Verbindungen eine hohe Fluoreszenzquantenausbeute, eine hohe relative Fluoreszenz aus Wellenleitung und einen hohen Stokes-Shift aufweisen. Die Verbindungen sollen auch einen hohen Ordnungsgrad in flüssigkristallinen Mischungen aufweisen.

Die Verbindungen und Vorrichtungen sollen auch eine hohe Lebensdauer und einen hohen Schalthub aufweisen (d. h. Unterschieds der Transmission im Hellzustand zum Dunkelzustand). Weiterhin besteht bei Vorrichtungen, welche die Fluoreszenzemission der Farbstoffe zur Wiedergewinnung von Energie mittels einer Solarzelle nutzen, Verbesserungspotential bezüglich der Energieausbeute. Im optimalen Fall sollte die durch die Solarzelle bereitgestellte Energie ausreichen, um die gesamte für den Betrieb der Vorrichtung benötigte Energie bereitzustellen, bzw. sogar über diese Menge hinausgehen.

Die Verbindungen sollen auch für andere Anwendungen geeignet sein, beispielsweise organische Solarzellen, Flüssigkristallanzeigen und organische Elektronikbauteile, wie Halbleiter, Dioden oder OLEDs.

Überraschend wurde nun gefunden, dass die genannten technischen Aufgaben durch Verbindungen und Vorrichtungen gemäß den Patentansprüchen gelöst werden.

Gegenstand der Erfindung sind Verbindungen der Formel (I): 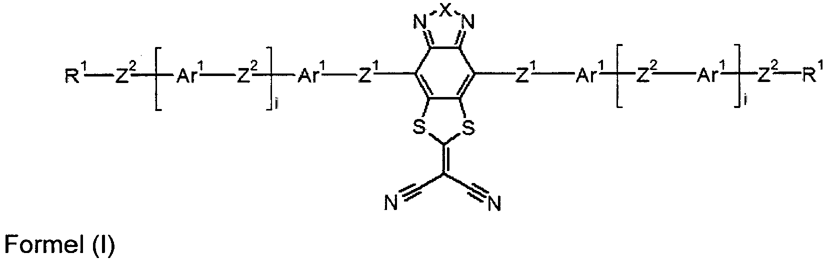 wobei gilt:
- X: ist gleich S oder Se;
- Z¹: ist unabhängig voneinander eine Einfachbindung, -CR³=CR³- oder -C≡C-; oder zwei, drei, vier oder fünf miteinander kombinierte Gruppen, gewählt aus den Gruppen -CR³=CR³- und -C≡C-;
- Z²: ist unabhängig voneinander eine Einfachbindung, O, S, C(R³)₂, -CR³=CR³- oder -C≡C-; oder zwei, drei, vier oder fünf miteinander kombinierte Gruppen, gewählt aus den Gruppen O, S, C(R³)₂, -CR³=CR³- und -C≡C-;
- Ar¹: ist unabhängig voneinander eine Aryl- oder Heteroarylgruppe mit 5 bis 30 aromatischen Ringatomen, die mit einem oder mehreren Resten R⁴ substituiert sein kann;
- R¹: ist unabhängig voneinander H, D, F, CN, N(R⁵)₂, oder eine Alkyl-, Alkoxy- oder Thioalkoxygruppe mit 1 bis 20 C-Atomen, die mit einem oder mehreren Resten R⁵ substituiert sein kann, wobei eine oder mehrere CH₂-Gruppen in den Alkyl-, Alkoxy- oder Thioalkoxygruppen durch -R⁵C=CR⁵⁻, -C≡C-, C=O, C=S, -C(=O)O-, -OC(=O)-, Si(R⁵)₂, NR⁵, -O- oder -S- ersetzt sein können;
- R³, R⁴: sind unabhängig voneinander H, D, F, Cl, CN, oder eine Alkyl-, Alkoxy-, oder Thioalkoxygruppe mit 1 bis 10 C-Atomen, die mit einem oder mehreren Resten R⁵ substituiert sein kann, wobei eine oder mehrere CH₂-Gruppen in den Alkyl-, Alkoxy- oder Thioalkoxygruppen durch -R⁵C=CR⁵⁻, -C≡C-, C=O, C=S, -C(=O)O-, -OC(=O)-, Si(R⁵)₂, NR⁵, -O- oder -S- ersetzt sein können;
- R⁵: ist unabhängig voneinander H, D, F, Cl, CN, N(R⁶)₂, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, C=O, C=S, -C(=O)O-, -O(C=O)-, Si(R⁶)₂, NR⁶, -O- oder -S- ersetzt sein können, oder eine Aryl- oder Heteroarylgruppe mit 5 bis 30 aromatischen Ringatomen, die jeweils mit einem oder mehreren Resten R⁶ substituiert sein kann;
- R⁶: ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können;
- i: ist unabhängig voneinander gleich 0, 1, 2, 3, 4 oder 5.

Wenn i größer als 1 ist, können die Gruppen innerhalb der Klammern gleich oder verschieden sein.

Wenn i gleich 0 ist, fällt die Gruppe innerhalb der Klammern weg, und die Gruppen Ar¹ und Z² sind direkt miteinander verbunden.

Die Verbindung der Formel (I) ist ein Farbstoff. Dies bedeutet, dass sie im sichtbaren Bereich Licht zumindest teilweise absorbiert. Die Verbindung ist bevorzugt dichroitisch. Dies bedeutet, dass sie Licht in Abhängigkeit von der Polarisation unterschiedlich stark absorbiert. Die Verbindung ist insbesondere ein dichroitischer Farbstoff.

Die Verbindungen der Formel (I) weisen eine zentrale Untereinheit 2-(2,5,7-Trithia-1,3-diaza-s-indacen-6-yliden)malononitril (TDIM) auf, die in Formel (I) zwischen den beiden Resten Z¹ dargestellt ist. Diese Struktureinheit weist drei anellierte Ringe auf, die ein heterocyclisches Derivat von Indacen bilden. Das Schwefel-Atom an der C2 Position kann dabei, wie in Formel (I) dargestellt, durch Selen ersetzt sein.

Unter der Formulierung "zwei, drei, vier oder fünf miteinander kombinierte Gruppen, gewählt aus den Gruppen..." ist im Sinne der vorliegenden Anmeldung zu verstehen, dass die Gruppen aneinander gebunden vorliegen, bevorzugt in Form einer Kette, in der zwei, drei, vier oder fünf der Gruppen aneinander gebunden vorliegen. Bevorzugt ist eine Kombination von genau zwei oder drei Gruppen. Die Gruppen können allgemein gleich oder verschieden sein.

Die Verbindungen der Formel (I) weisen Vorteile gegenüber dem Stand der Technik auf und lösen dadurch die der Erfindung zugrunde liegende Aufgabe. Die Verbindungen zeigen in Lösung in einem flüssigkristallinen Host eine extreme Lichtechtheit, z. B. im Vergleich zu Perylendiimid-Derivaten. Außerdem zeigen sie eine langwellige Absorption, und hohen Dichroismus in Kombination mit sehr guter Löslichkeit. Ein besonderer Vorteil ist auch die Möglichkeit, über eine Variation der Elektron-Donor-Eigenschaften der Seitenketten, die Absorptions- und Emissionsmaxima auf ihre jeweiligen Anforderungen einzustellen. Die Verbindungen weisen auch einen hohen Ordnungsgrad in flüssigkristallinen Mischungen auf, der im allgemeinen höher ist als der von Bezothiadiazol-Derivaten.

Ein wesentlicher Vorteil ist die Lage des Fluoreszenz-Emissionsmaximums jenseits des Empfindlichkeitsbereichs des menschlichen Auges. In Anwendungen, wie beispielsweise schaltbaren Fenstern auf Basis flüssigkristalliner Guest-Host-Systeme, ermöglicht dies die Nutzung der emittierten Strahlung für die Elektrizitätsversorgung energieautarker Systeme mit Hilfe von Solarzellen, ohne dass das für das Auge unsichtbare Fluoreszenzlicht zu irritierenden Störeffekten bei solchen Fenstersystemen führt.

Bevorzugt weist die Verbindung der Formel (I) an jeder Seite der zentralen Einheit zwei oder drei aromatische Gruppen auf. Bevorzugt bilden die aromatischen Gruppen der Verbindung von Formel (I) eine Kette.

In der Formel (I) können die gezeigten Gruppen allgemein gleich oder verschieden voneinander sein. Wenn also eine Verbindung der Formel (I) zwei oder mehr Gruppen aufweist, die in der Formel mit identischen Platzhaltern bezeichnet sind, wie beispielsweise Ar¹, so können die zwei oder mehr Gruppen Ar¹ gleich oder verschieden voneinander sein.

Eine Arylgruppe im Sinne dieser Erfindung enthält 6 bis 30 aromatische Ringatome; eine Heteroarylgruppe im Sinne dieser Erfindung enthält 5 bis 30 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (anellierter) aromatischer bzw. heteroaromatischer Polycyclus, beispielsweise Naphthalin, Phenanthren, Chinolin oder Carbazol verstanden. Ein kondensierter (anellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen. Ein solcher Polycyclus kann auch einzelne nichtkonjugierte Einheiten enthalten, wie es beispielsweise beim Fluoren-Grundkörper der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Fluoren, Spirobifluoren, Furan, Benzofuran, Isobenzofuran, Dibenzofuran, Thiophen, Benzothiophen, Isobenzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Bevorzugt sind die Heteroarylgruppen der Verbindung (I) verschieden von der zentralen TDIM Gruppe.

Im Rahmen der vorliegenden Erfindung werden unter einer Alkylgruppe mit 1 bis 10 C-Atomen bzw. einer Alkenyl- oder Alkinylgruppe mit 2 bis 10 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkoxygruppe mit 1 bis 10 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter einem aliphatischen organischen Rest mit 1 bis 20 C-Atomen wird prinzipiell ein beliebiger organischer Rest verstanden, welcher nicht aromatisch bzw. heteroaromatisch ist. Bevorzugt werden darunter Alkylgruppen mit 3 bis 20 oder 4 bis 15 C-Atomen, Alkoxygruppen mit 3 bis 20 oder 4 bis 15 C-Atomen, Alkenyl- oder Alkinylgruppen mit 2 bis 10 C-Atomen, wie oben näher beschrieben, verstanden.

Es ist bevorzugt, dass X für S steht. Die zentrale Gruppe in Formel (I) ist dann eine TDIM-Struktureinheit. Im Rahmen dieser Anmeldung wird der Begriff TDIM als Abkürzung für 2-(2,5,7-Trithia-1,3-diaza-s-indacen-6-yliden)malononitril, wie in Formel 1 gezeigt, verwendet.

Das Schwefel-Atom an der C2 Position kann bei allen nachfolgend beschriebenen Verbindungen durch Selen ersetzt sein, wie in Formel (I) anhand des Substituenten X dargestellt. Bevorzugt ist dies nicht der Fall.

Für Z¹ ist es bevorzugt, dass es bei jedem Auftreten gleich oder verschieden für eine Einfachbindung, -CR³=CR³- oder -C≡C- steht. Besonders bevorzugt ist Z¹ eine Einfachbindung.

Für Z² ist es bevorzugt, dass es bei jedem Auftreten gleich oder verschieden für eine Einfachbindung, -C(R³)₂C(R³)₂-, -CR³=CR³-, -C≡C-, -OC(R³)₂- oder -C(R³)₂O- steht, besonders bevorzugt für eine Einfachbindung, -CH₂CH₂-, -CF₂CF₂-, -CH=CH-, -CF=CF-, -C≡C-, -OCH₂-, -OCF₂-, -CH₂O- oder -CF₂O-. Besonders bevorzugt ist Z² eine Einfachbindung.

Für Ar¹ ist es bevorzugt, dass es bei jedem Auftreten gleich oder verschieden eine Arylgruppe mit 6 bis 15 C-Atomen oder eine Heteroarylgruppe mit 5 bis 15 C-Atomen darstellt, die mit einem oder mehreren Resten R⁴ substituiert sein kann. Besonders bevorzugt ist Ar¹ bei jedem Auftreten gleich oder verschieden gewählt aus wahlweise mit Resten R⁴ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin. Besonders bevorzugt ist Benzol oder Thiophen, die gegebenenfalls mit Fluor, bevorzugt einem oder zwei Fluor pro Ring, substituiert sein können.

Die Gruppe R¹ ist bevorzugt bei jedem Auftreten gleich oder verschieden H, F, CN, N(R⁵)₂, oder eine geradkettige Alkyl- oder Alkoxygruppe mit 1 bis 20 C-Atomen, die mit einem oder mehreren Resten R⁵ substituiert sein kann, oder eine verzweigte Alkyl- oder Alkoxygruppe mit 3 bis 20 C-Atomen, die mit einem oder mehreren Resten R⁵ substituiert sein kann, oder eine cyclische Alkylgruppe mit 4 bis 8 C-Atomen, die mit einem oder mehreren Resten R⁵ substituiert sein kann, wobei in den Alkyl- und Alkoxygruppen eine oder mehrere CH₂-Gruppen durch -O-, -S- oder -R⁵C=CR⁵- ersetzt sein können, oder eine Siloxanylgruppe mit 1 bis 10 Si-Atomen, die mit einem oder mehreren Resten R⁵ substituiert sein kann.

Besonders bevorzugt ist R¹ unabhängig voneinander ausgewählt aus H, F, oder einer geradkettigen Alkyl- oder Alkoxylgruppe mit 3 bis 20 C-Atomen, die mit einem oder mehreren Resten R⁵ substituiert sein kann, oder einer verzweigten Alkyl- oder Alkoxygruppe mit 3 bis 20 C-Atomen, die mit einem oder mehreren Resten R⁵ substituiert sein kann, oder einer cyclischen Alkylgruppe mit 6 C-Atomen, die mit einem oder mehreren Resten R⁵ substituiert sein kann, wobei in den Alkyl- und Alkoxygruppen eine oder mehrere CH₂-Gruppen durch -O-, -S- oder -R⁵C=CR⁵- ersetzt sein können, oder einer Siloxanylgruppe mit 1 bis 6 Si-Atomen, die mit einem oder mehreren Resten R⁵ substituiert sein kann.

Ganz besonders bevorzugt ist/sind mindestens eine Seitenkette R¹, bevorzugt beide Seitenketten R¹, ausgewählt aus geradkettigen Alkyl- oder Alkoxylgruppen mit 3 bis 20, insbesondere 4 bis 15 C-Atomen, oder verzweigten Alkyl- oder Alkoxygruppen mit 3 bis 20, insbesondere 4 bis 15 C-Atomen, oder cyclischen Alkylgruppen mit 6 C-Atomen, oder N(R¹⁰)₂-Gruppen, wobei R¹⁰ unabhängig voneinander ausgewählt ist aus Alkyl mit 1 bis 10 C Atomen. Eine verzweigte Kette R¹ weist bevorzugt mindestens ein Chiralitätszentrum auf.

Insbesondere bevorzugt sind beide Seitenketten R¹ Alkylgruppen mit mindestens 2, bevorzugt 3 bis 20, insbesondere 4 bis 15 C-Atomen, bevorzugt verzweigt, bevorzugt mit einem Chiralitätszentrum.

R³ ist bevorzugt bei jedem Auftreten gleich oder verschieden H, F, oder eine Alkylgruppe mit 1 bis 10 C-Atomen, die mit einem oder mehreren Resten R⁵ substituiert sein kann. Besonders bevorzugt ist R³ bei jedem Auftreten gleich oder verschieden H oder F.

R⁴ ist bevorzugt bei jedem Auftreten gleich oder verschieden H, D, F, CN, oder eine Alkyl- oder Alkoxygruppe mit 1 bis 10 C-Atomen, die mit einem oder mehreren Resten R⁵ substituiert sein kann. Besonders bevorzugt ist R³ bei jedem Auftreten gleich oder verschieden H oder F.

R⁵ ist bei jedem Auftreten gleich oder verschieden H, F, CN, oder eine Alkyl- oder Alkoxygruppe mit 1 bis 10 C-Atomen, die mit einem oder mehreren Resten R⁶ substituiert sein kann, oder eine Aryl- oder Heteroarylgruppe mit 5 bis 30 aromatischen Ringatomen, die jeweils mit einem oder mehreren Resten R⁶ substituiert sein kann, oder eine Siloxanylgruppe mit 1 bis 6 Si-Atomen, die mit einem oder mehreren Resten R⁶ substituiert sein kann.

Für den Index i gilt, dass er bevorzugt gleich 1, 2, oder 3 ist, besonders bevorzugt gleich 1 oder 2, ganz besonders bevorzugt gleich 1.

Die Verbindungen der Formel (I) sind keine Polymere. Sie wurden nicht mittels Polymerisation hergestellt. Sie unterscheiden sich dadurch maßgeblich von im Stand der Technik beschriebenen dichroitischen Farbstoffen für die Photo- und Elektrochemie, die im allgemeinen Polymere sind.

Bevorzugt weisen die Verbindungen der Formel (I) insgesamt 3 bis 5 aromatische Ringstrukturen auf. Mit aromatischer Ringstruktur wird damit ein Ring, oder ein anelliertes Ringsystem bezeichnet, das bevorzugt 2 bis 4 anellierte Ringe aufweist. Besonders bevorzugt weist die Verbindung der Formel (I) 5 Ringsysteme auf, nämlich eine zentrale TDIM-Gruppe und jeweils 2 seitliche miteinander verbundene aromatische Ringe. Die aromatischen Ringstrukturen sind bevorzugt über C-C Einfachbindungen miteinander verknüpft.

Bevorzugt weisen die Reste R¹ jeweils mindestens 2 C-Atome, insbesondere jeweils mindestens eine Alkylkette mit mindestens 2, bevorzugt mit 3 bis 20 C-Atomen auf.

Besonders bevorzugt weisen die Verbindungen der Formel (I) insgesamt 3 bis 5 aromatische Ringstrukturen auf, und die Reste R¹ weisen jeweils mindestens eine Alkylkette mit mindestens 2 C-Atomen auf.

In einer bevorzugten Ausführungsform ist die Verbindung der Formel (I) eine chirale Verbindung. Sie wird bevorzugt als Racemat oder als Gemisch von Stereoisomeren (*d, I* oder *meso*) eingesetzt. Bevorzugt weist die Verbindung der Formel (I) mindestens eine, besonders bevorzugt eine oder zwei, verzweigte Seitenketten R¹ auf, die ein Chiralitätszentrum aufweisen.

In einer bevorzugten Ausführungsform ist die Verbindung der Formel (I) asymmetrisch, in dem Sinne, dass die beiden an die zentrale TDIM-Gruppe gebundenen Einheiten nicht identisch sind. Solche Verbindungen weisen oft besondere elektronische Eigenschaften auf, insbesondere besondere Fluoreszenzeigenschaften.

In einer bevorzugten Ausführungsform weist die Verbindung der Formel (I) bei <200°C Mesophasen auf.

Bevorzugte Ausführungsformen der Formel (I) entsprechen den folgenden Formeln (Ia) und (Ib): 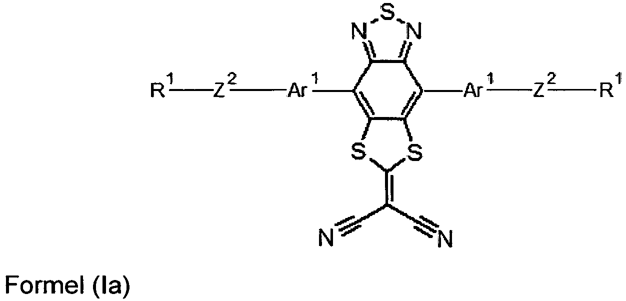 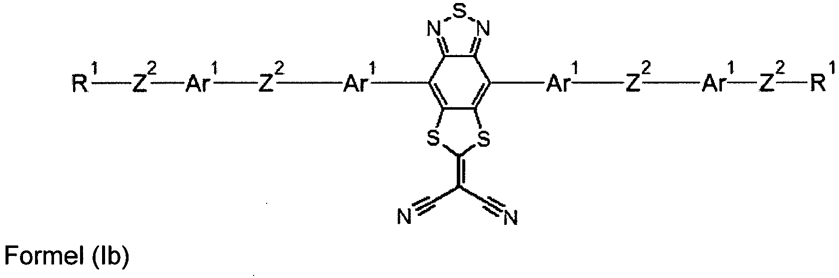 wobei die auftretenden Gruppen wie oben definiert sind.

Bevorzugt gelten für Formeln (Ia) und (Ib) die oben angegebenen bevorzugten Ausführungsformen der Gruppen Ar¹, Z² und R¹.

Erfindungsgemäß und insbesondere für die Formeln (Ia) und (Ib) ist es bevorzugt, dass mindestens ein direkt an die TDIM Struktureinheit gebundenes Ar¹ für eine schwefelhaltige Heteroarylgruppe steht, besonders bevorzugt für Thiophen. Die Gruppe kann mit einem oder mehreren Resten R⁴ substituiert sein. Derartige Verbindungen zeichnen sich durch eine besonders hohe Lichtstabilität aus.

In einer bevorzugten Ausführungsform sind alle Reste Z¹ und Z² Einfachbindungen. Dann weist die Verbindung der Formel (I) die Formel (Ic) auf: 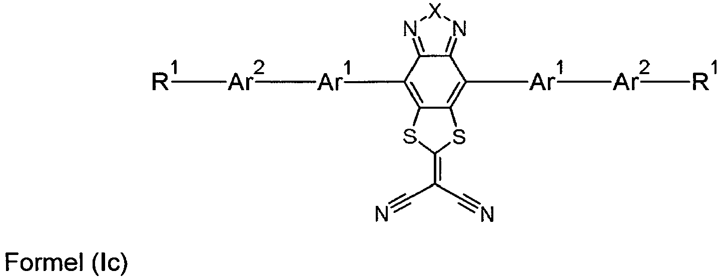 wobei die auftretenden Gruppen wie oben definiert sind. Bevorzugt weist die Formel (Ic) die oben angegebenen bevorzugten Gruppen Ar¹ und R¹ auf, wobei Ar² ausgewählt ist wie Ar¹.

Bevorzugte Ausführungsformen der Formeln (Ia) und (Ib) entsprechen den folgenden Formeln: 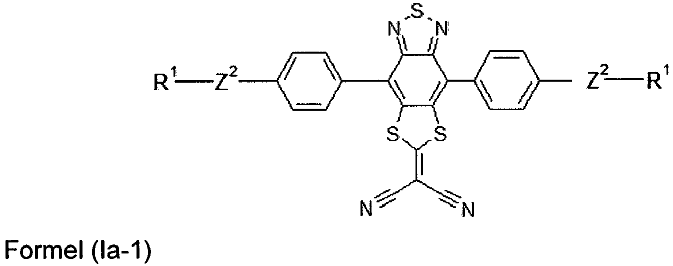 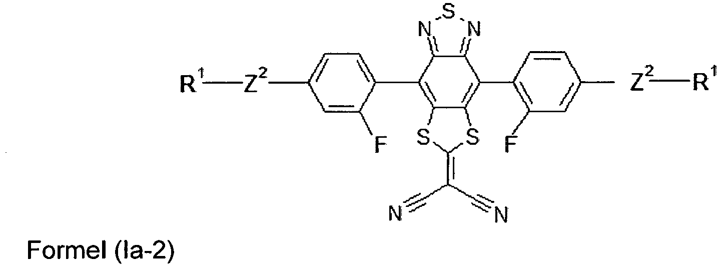 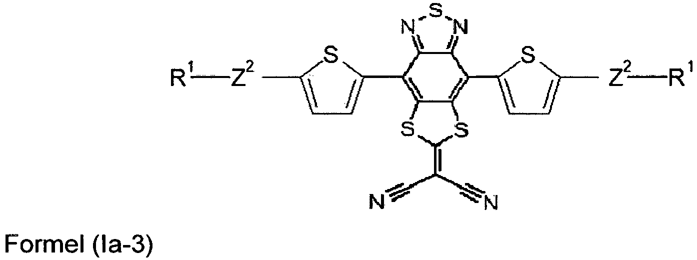 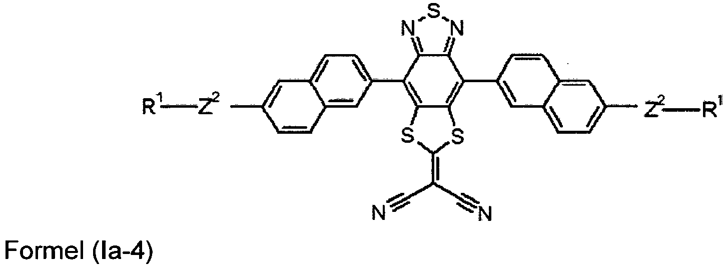 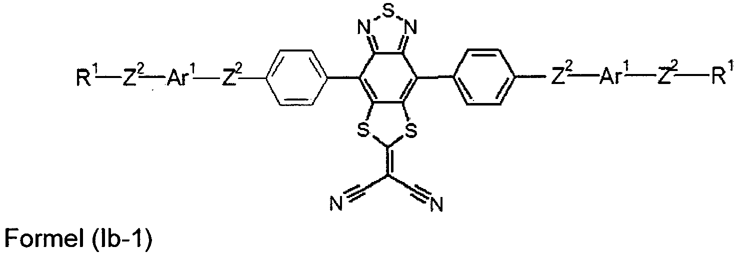 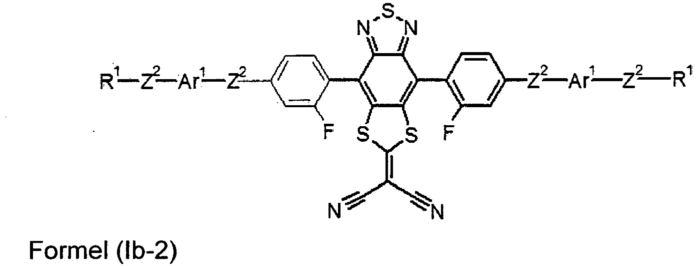 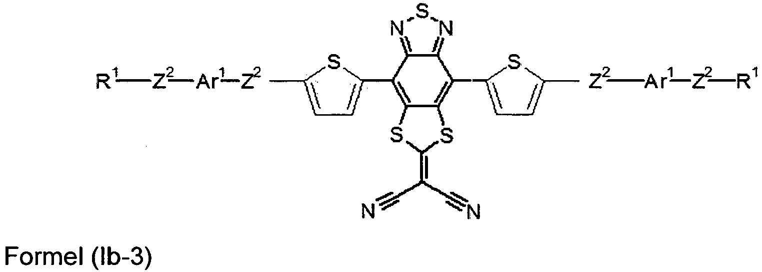 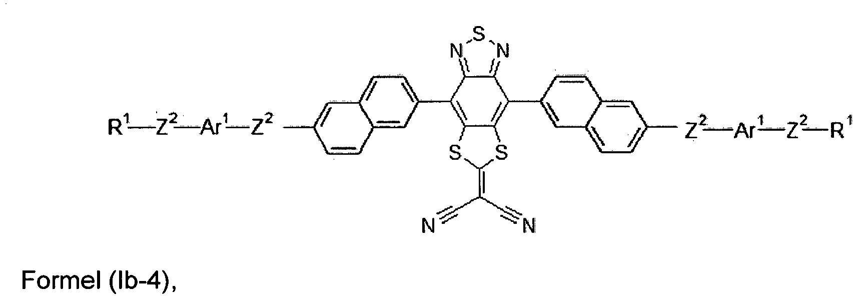 wobei die Gruppen Ar¹, Z² und R¹ definiert sind wie oben ausgeführt.

Besonders bevorzugte Verbindungen der Formel (I) sind die der folgenden Formel (IIa) bis (IIc): 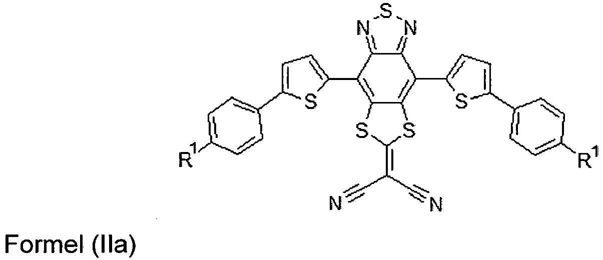 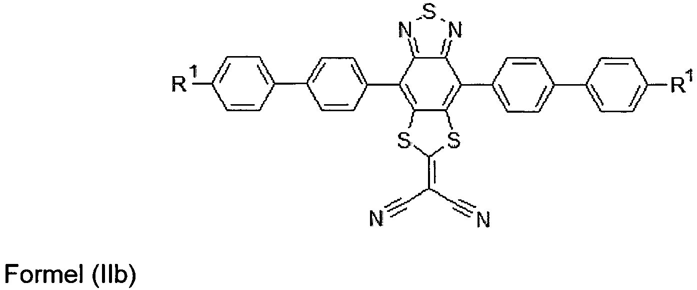 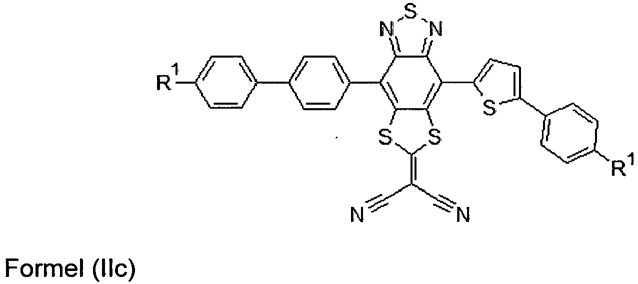 wobei die Reste R¹ unabhängig voneinander definiert sind wie oben ausgeführt. Die Benzol und Thiophenringe können fluoriert sein. Bevorzugt ist dabei nicht mehr als ein Fluor pro Ring vorhanden.

Weiterer Gegenstand der vorliegenden Erfindung sind Verbindungen gemäß Formel (I), die den folgenden Formeln (III) und (IV) entsprechen 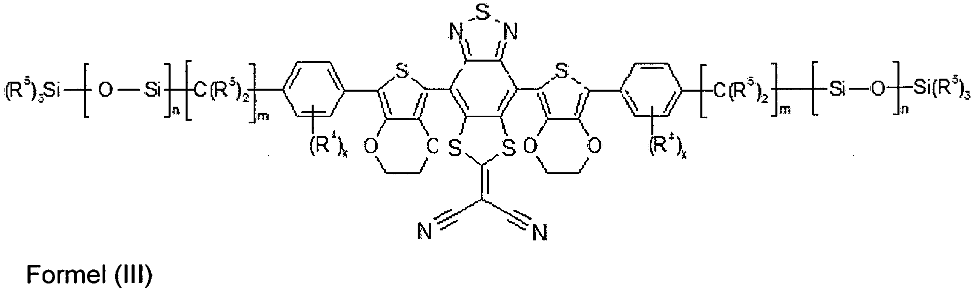 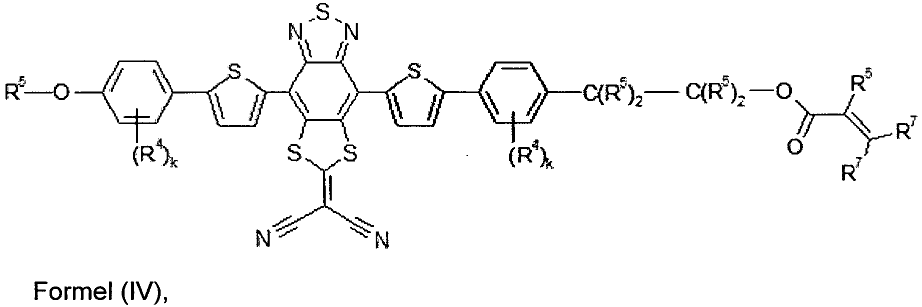 wobei gilt:
die auftretenden Gruppen R⁴ und R⁵ sind definiert wie oben, R⁷ ist definiert wie R⁵ oben, und
k ist, gleich oder verschieden bei jedem Auftreten, 0, 1, 2, 3 oder 4;
m ist, gleich oder verschieden bei jedem Auftreten, 0, 1, 2, 3, 4, 5 oder 6;
n ist, gleich oder verschieden bei jedem Auftreten, 1, 2, 3, 4 oder 5.

In einer bevorzugten Ausführungsform ist der Index k in den Formeln (III) oder (IV) gleich oder verschieden 0 oder 1, besonders bevorzugt gleich 0.

In einer bevorzugten Ausführungsform ist der Index m in den Formeln (III) oder (IV) gleich oder verschieden 0, 1 oder 2, besonders bevorzugt gleich 1 oder 2.

In einer bevorzugten Ausführungsform ist der Index n in den Formeln (III) oder (IV) gleich oder verschieden 1, 2 oder 3, besonders bevorzugt gleich 2.

Weiterhin bevorzugt ist R⁵ in den Formeln (III) oder (IV) Wasserstoff oder eine Alkylgruppe mit 1 bis 5 C-Atomen, die mit einem oder mehreren Resten R⁶ substituiert sein kann, besonders bevorzugt Methyl.

In einer bevorzugten Ausführungsform ist R⁷ in der Formel (III) oder (IV) Wasserstoff oder eine Alkylgruppe mit 1 bis 5 C-Atomen, die mit einem oder mehreren Resten R⁶ substituiert sein kann, besonders bevorzugt Wasserstoff.

Die Verbindungen der Formel (III) und (IV) weisen besonders große Vorteile in Bezug auf die oben genannten Eigenschaften gute Löslichkeit im flüssigkristallinen Medium, gute Lichtstabilität, hohe Fluoreszenz und/oder hohe Anisotropie der Absorption bzw. dichroitisches Verhältnis auf.

Bevorzugte Verbindungen sind solche der folgenden Formeln V1 bis V15, wobei die Verbindungen V1 bis V6 besonders bevorzugt sind:

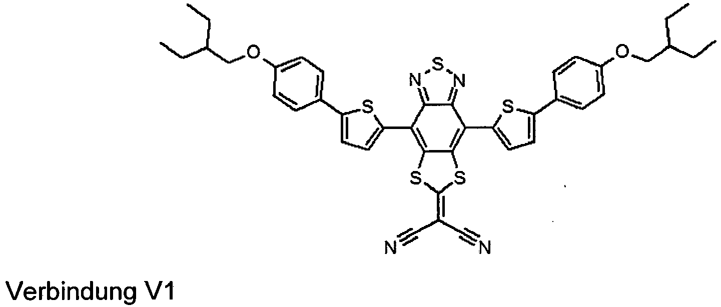

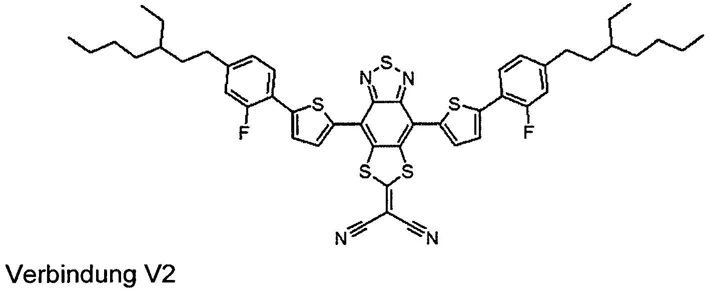

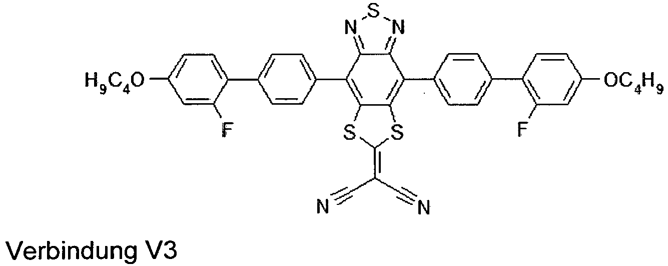

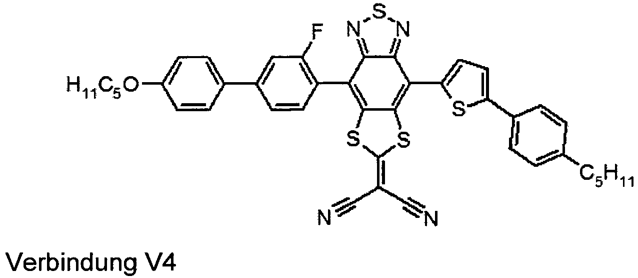

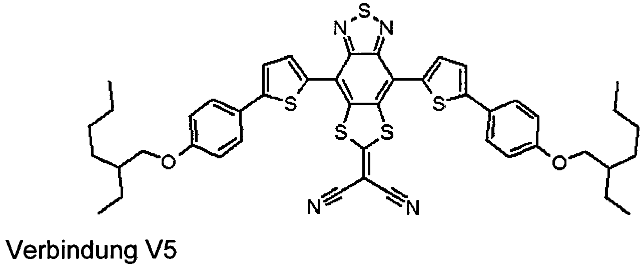

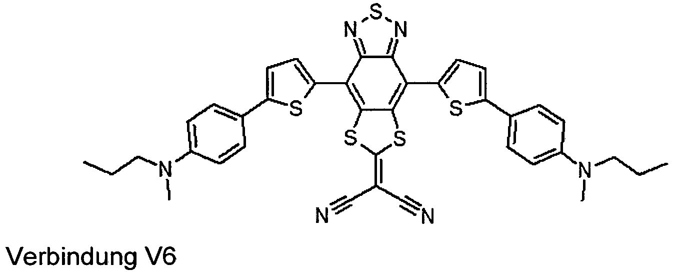

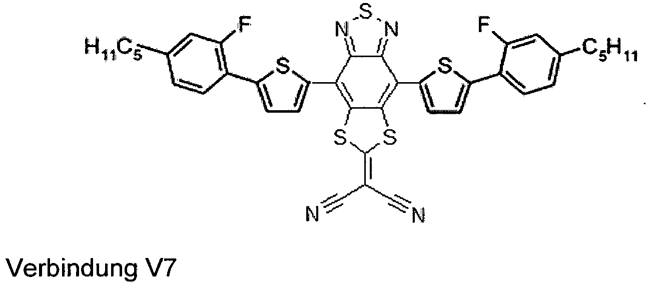

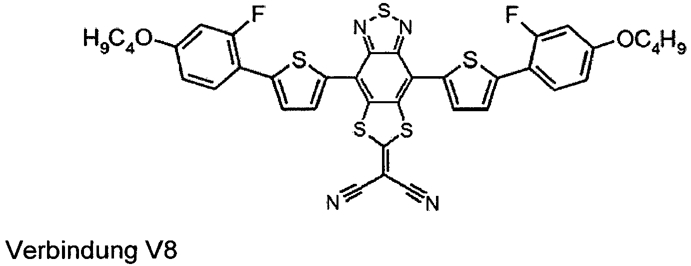

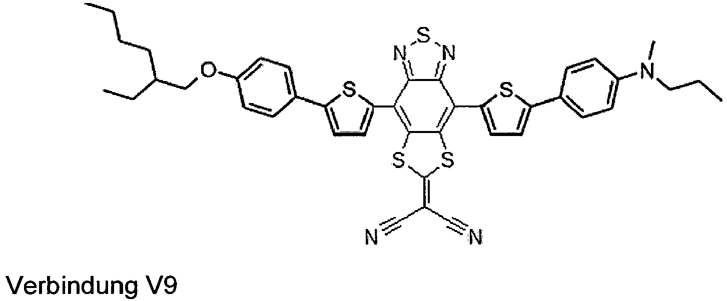

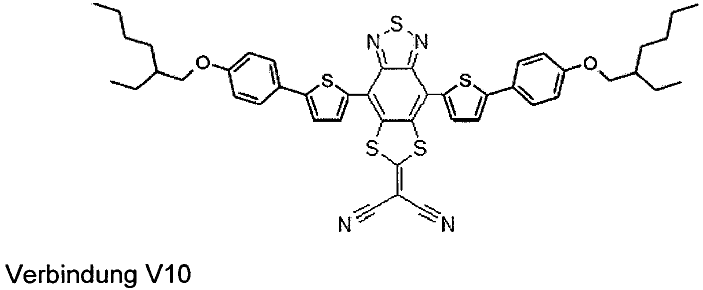

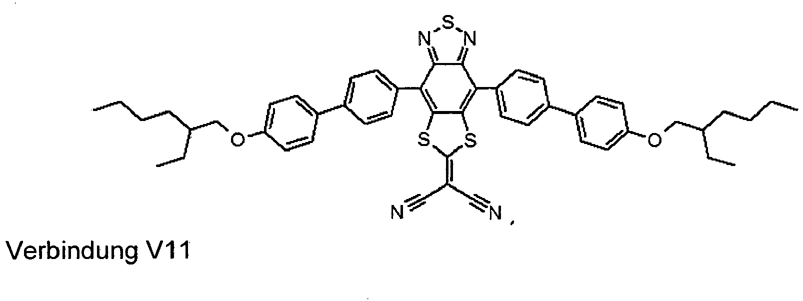

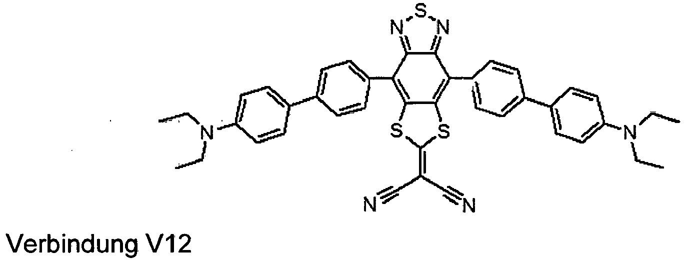

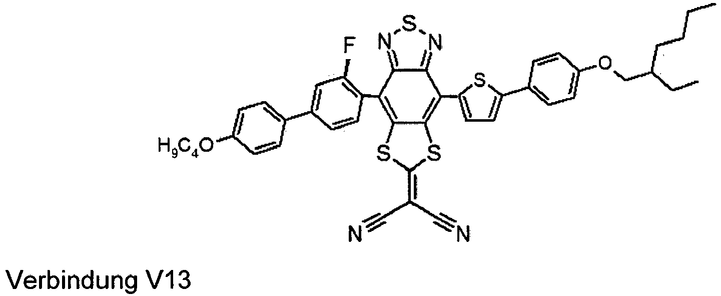

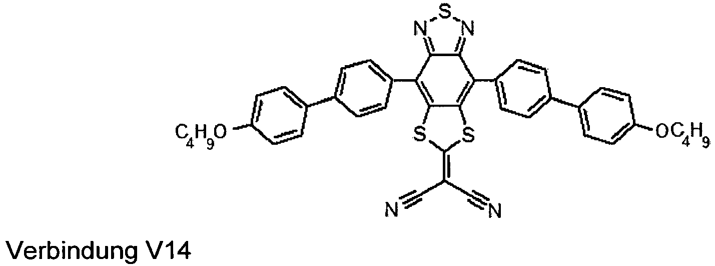

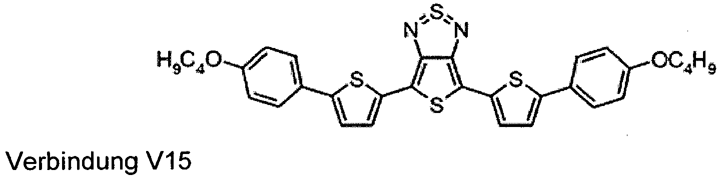

Die Verbindungen der Formel (I) können gemäß grundsätzlich bekannten Verfahren der organischen Chemie hergestellt werden, insbesondere durch Suzuki-Kupplung zwischen organischen Bromiden und organischen Boronsäuren, oder durch Stille-Kopplung über die Tributylstannyl-Derivate. Im Folgenden werden besonders geeignete Verfahren in allgemeiner Form genannt. Für konkrete Verfahren zur Herstellung von Verbindungen der Formel (I) wird weiterhin auf die bekannte Literatur und auf die Ausführungsbeispiele verwiesen.

In einer bevorzugten Ausführungsform werden die Verbindungen ausgehend von Dihalogendifluorbenzothiadiazol, insbesondere Dibromdifluorbenzothiadiazol, hergestellt.

Die folgenden allgemeine Syntheseschemata 1 bis 3 zeigen beispielhaft, wie Verbindungen der Formel (I) herzustellen sind, insbesondere Verbindungen der Formel (IIa), (IIb) und (IIc). Die Synthesen gehen von Dihalogendifluorbenzothiadiazol, insbesondere Dibromdifluorbenzothiadiazol aus. Die Herstellung solcher Verbindungen wird in Zhang et al., 2011, und Li et al., 2014, beschrieben. Die Kopplungen erfolgen nach Standardmethoden über die Boronsäure- (Suzuki-Kopplung) oder die Tributylstannyl-Derivate (Stille-Kopplung). In den Schemata entspricht Rest R dem Rest R¹ von Formel (I), NBS steht für N-Bromsuccinimid, NMP steht für N-Methyl-2-pyrrolidon.

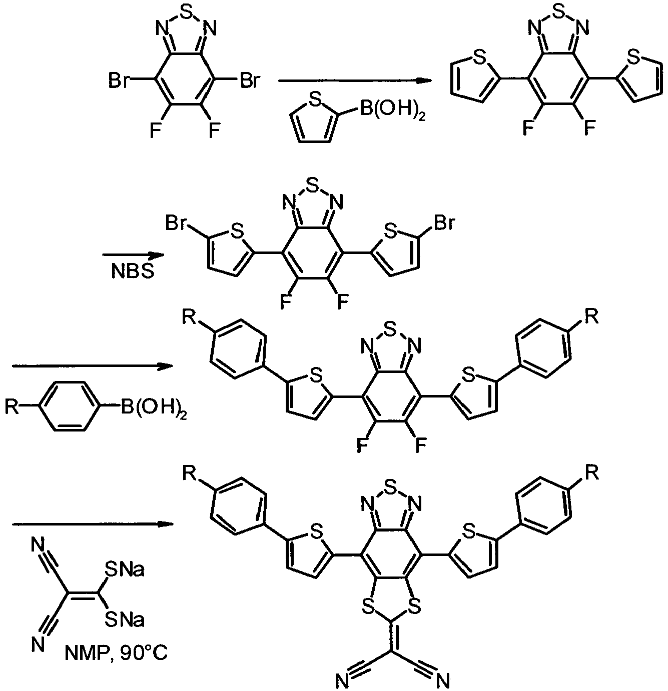

### Schema 1: Synthese von Verbindungen der Formel (IIa)

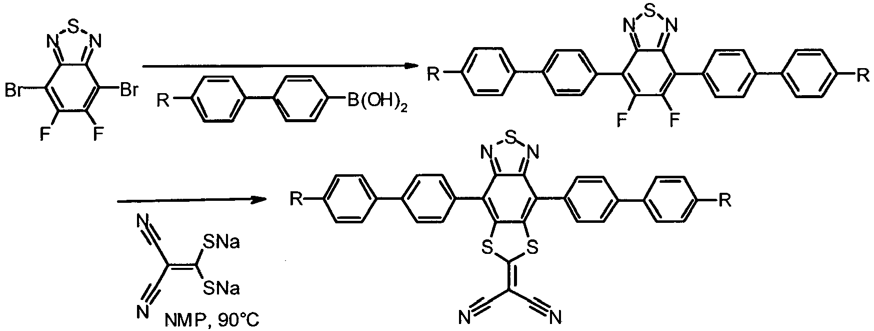

### Schema 2: Synthese von Verbindungen der Formel (IIb)

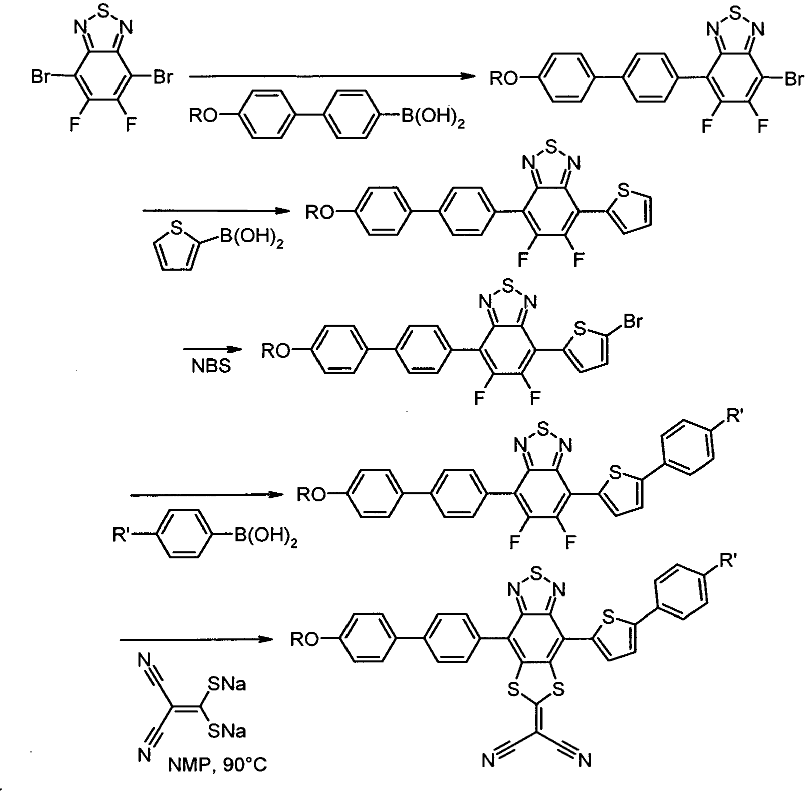

### Schema 3: Synthese von Verbindungen der Formel (IIc)

Gegenstand der Erfindung ist auch eine Vorrichtung, enthaltend mindestens eine der erfindungsgemäßen Verbindungen.

Die Vorrichtung ist insbesondere ausgewählt aus Fenstern, insbesondere einem schaltbaren Fenster, organischen Solarzellen, insbesondere Bulk-Hetero-Junction Solarzellen, Flüssigkristallanzeigen (Guest Host LCD), Halbleitern (Donor oder Akzeptor), organischen Elektronikbauteilen, wie Feldeffekttransistoren, Dioden oder OLEDs. Die Farbstoffe können auch zum Anfärben einer Polymermatrix eingesetzt werden, beispielsweise im Automobilbereich.

Die Verbindung der Formel (I) ist bevorzugt ein positiv dichroitischer Farbstoff, d. h. ein Farbstoff, welcher einen positiven Anisotropiegrad R aufweist. Besonders bevorzugt ist der Anisotropiegrad R größer als 0.4, ganz besonders bevorzugt größer als 0.6 und am stärksten bevorzugt größer als 0.7. Der Anisotropiegrad kann beispielsweise ermittelt werden wie in dem Ausführungsbeispiel der EP14002950.5, eingereicht am 25.08.2014, angegeben.

Bevorzugt erreicht die Absorption ein Maximum, wenn die Polarisationsrichtung des Lichts parallel zur Richtung der längsten Ausdehnung des Moleküls gemäß Formel (I) ist, und sie erreicht ein Minimum, wenn die Polarisationsrichtung des Lichts senkrecht zur Richtung der längsten Ausdehnung des Moleküls gemäß Formel (I) ist.

Die Verbindungen der Formel (I) weisen bevorzugt eine geringe Bandlücke und eine gute Absorption oberhalb 450 nm auf. Bevorzugt weisen sie eine Absorptionsbande oberhalb 450 nm auf.

Weiterhin bevorzugt ist die Verbindung der Formel (I) ein fluoreszierender Farbstoff. Unter Fluoreszenz wird dabei verstanden, dass eine Verbindung durch Absorption von Licht einer bestimmten Wellenlänge in einen elektronisch angeregten Zustand versetzt wird, wobei die Verbindung anschließend unter Emission von Licht in den Grundzustand übergeht. Bevorzugt hat das emittierte Licht längere Wellenlänge als das absorbierte Licht. Weiterhin bevorzugt ist der Übergang vom angeregten Zustand in den Grundzustand Spin-erlaubt, erfolgt also ohne Änderung des Spins. Weiterhin bevorzugt ist die Lebensdauer des angeregten Zustands der fluoreszierenden Verbindung kürzer als 10⁻⁵ s, besonders bevorzugt kürzer als 10⁻⁶ s, ganz besonders bevorzugt zwischen 10⁻⁹ und 10⁻⁷ s.

Die dichroitische Verbindung der Formel (I) liegt bevorzugt in einem Anteil von 0.01 bis 10 Gew.-%, besonders bevorzugt 0.05 bis 7 Gew.-% und ganz besonders bevorzugt 0.1 bis 7 Gew.-% in der Schaltschicht vor.

Neben der Verbindung der Formel (I) liegt in der Schaltschicht bevorzugt ein flüssigkristallines Medium enthaltend eine oder mehrere verschiedene Verbindungen vor. Bevorzugt stellt das flüssigkristalline Medium die Hauptkomponente der Mischung der Schaltschicht der Vorrichtung dar. Die dichroitische Verbindung der Formel (I) liegt in der Schaltschicht bevorzugt in Lösung vor. Sie wird bevorzugt in ihrer Ausrichtung durch die Ausrichtung der Verbindungen des flüssigkristallinen Mediums beeinflusst.

Unter dem Begriff flüssigkristallines Medium wird im Rahmen der vorliegenden Anmeldung ein Material verstanden, das unter bestimmten Bedingungen flüssigkristalline Eigenschaften aufweist. Bevorzugt hat das Material bei Raumtemperatur und in einer gewissen Temperaturspanne darüber und darunter flüssigkristalline Eigenschaften. Das flüssigkristalline Medium kann eine einzige Verbindung enthalten, oder es kann mehrere unterschiedliche Verbindungen enthalten. Typischerweise enthält das flüssigkristalline Medium gemäß der Erfindung mindestens eine Verbindung, deren Moleküle eine langgestreckte Form aufweisen, d.h. in einer Raumrichtung deutlich länger (Längsachse) sind als in den anderen beiden Raumrichtungen.

Weiterer Gegenstand der Erfindung ist die Verwendung einer Mischung, enthaltend ein flüssigkristallines Medium und mindestens eine Verbindung einer Formel (I), in einer Vorrichtung zur Regulierung des Energie-Durchtritts von einem Innenraum in einen Außenraum.

Bevorzugt hat das flüssigkristalline Medium der Schaltschicht einen Klärpunkt, bevorzugt einen Phasenübergang von einem nematisch flüssigkristallinen Zustand zu einem isotropen Zustand, im Temperaturbereich von 70 °C bis 170 °C, bevorzugt von 90 °C bis 160 °C, besonders bevorzugt von 95 °C bis 150 °C und ganz besonders bevorzugt von 105 °C bis 140 °C.

Bevorzugt ist weiterhin die dielektrische Anisotropie des flüssigkristallinen Mediums der Schaltschicht größer als 3, besonders bevorzugt größer als 7.

In einer weiteren bevorzugten Ausführungsform ist die dielektrische Anisotropie des flüssigkristallinen Mediums der Schaltschicht kleiner als null, besonders bevorzugt kleiner als -2.

Weiterhin bevorzugt hat das flüssigkristalline Medium der Schaltschicht eine optische Anisotropie (Δn) von 0.01 bis 0.3, besonders bevorzugt von 0.04 bis 0.27.

Weiterhin bevorzugt umfasst das flüssigkristalline Medium der Schaltschicht 3 bis 20 verschiedene flüssigkristalline Verbindungen, bevorzugt 8 bis 18, besonders bevorzugt 12 bis 16 verschiedene flüssigkristalline Verbindungen.

Verbindungen, welche als Bestandteile des flüssigkristallinen Mediums verwendet werden können, sind dem Fachmann bekannt und können beliebig gewählt werden.

Es ist bevorzugt, dass das flüssigkristalline Medium der Schaltschicht mindestens eine Verbindung enthält, welche Strukturelemente basierend auf 1,4-Phenylenen und 1,4-Cyclohexylenen aufweist, die mit einem oder mehreren Fluoratomen oder einer oder mehreren Nitrilgruppen substituiert sind. Besonders bevorzugt ist es, dass das flüssigkristalline Medium der Schaltschicht mindestens eine Verbindung enthält, welche 2, 3 oder 4, besonders bevorzugt 3 oder 4 Strukturelemente basierend auf 1,4-Phenylenen und 1,4-Cyclohexylenen aufweist.

Es ist weiterhin bevorzugt, dass das flüssigkristalline Medium der Schaltschicht einen oder mehrere chirale Dotierstoffe enthält. Bevorzugt liegen in diesem Fall in der Schaltschicht der Vorrichtung die Moleküle des flüssigkristallinen Medium gegeneinander verdrillt vor, besonders bevorzugt wie aus dem TN-Modus von Displays bekannt.

Chirale Dotierstoffe werden in dem flüssigkristallinen Medium der Schaltschicht bevorzugt in einer Gesamtkonzentration von 0.01 bis 3 Gew.-%, besonders bevorzugt von 0.05 bis 1 Gew.-% verwendet. Um hohe Werte für die Verdrillung zu erhalten, kann die Gesamtkonzentration der chiralen Dotierstoffe auch höher als 3 Gew.-% gewählt werden, bevorzugt bis maximal 10 Gew.-%.

Gemäß einer alternativen, ebenfalls bevorzugten Ausführungsform, enthält das flüssigkristalline Medium der Schaltschicht keine chiralen Dotierstoffe. Bevorzugt liegen in diesem Fall in der Schaltschicht die Moleküle des flüssigkristallinen Mediums nicht gegeneinander verdrillt vor.

Die Anteile dieser Verbindungen und anderer Mindermengenkomponenten werden bei der Angabe der Anteile der flüssigkristallinen Verbindungen und der dichroitischen Farbstoffe vernachlässigt.

Das flüssigkristalline Medium der Schaltschicht enthält weiterhin bevorzugt einen oder mehrere Stabilisatoren. Die Gesamtkonzentration der Stabilisatoren liegt bevorzugt zwischen 0.00001 und 10 Gew.-%, besonders bevorzugt zwischen 0.0001 und 1 Gew.-% der Gesamtmischung. Die Anteile dieser Verbindungen und anderer Mindermengenkomponenten werden bei der Angabe der Anteile der flüssigkristallinen Verbindungen und der dichroitischen Farbstoffe vernachlässigt.

Die erfindungsgemäße Vorrichtung enthält bevorzugt in der Schaltschicht zusätzlich zu einer oder mehreren Verbindungen der Formel (I), und bevorzugt einem flüssigkristallinen Medium, noch weitere dichroitische Farbstoffe mit anderer Struktur als Formel (I). Besonders bevorzugt enthält es einen, zwei, drei oder vier weitere Farbstoffe, ganz besonders bevorzugt zwei oder drei weitere Farbstoffe und am stärksten bevorzugt drei weitere Farbstoffe mit anderer Struktur als Formel (I).

Bezüglich der Eigenschaft des Dichroismus gelten die für die Verbindung der Formel (I) beschriebenen bevorzugten Eigenschaften auch für die optionalen weiteren dichroitischen Farbstoffe als bevorzugt.

Die Absorptionsspektren der dichroitischen Farbstoffe der Schaltschicht ergänzen sich bevorzugt derart, dass für das Auge der Eindruck von schwarzer Farbe entsteht. Bevorzugt decken die zwei oder mehr dichroitischen Farbstoffe des erfindungsgemäßen flüssigkristallinen Mediums einen großen Teil des sichtbaren Spektrums ab. Wie genau eine Mischung von Farbstoffen hergestellt werden kann, die für das Auge schwarz bzw. grau erscheint, ist dem Fachmann bekannt und beispielsweise in Manfred Richter, Einführung in die Farbmetrik, 2. Auflage, 1981, ISBN 3-11-008209-8, Verlag Walter de Gruyter & Co., beschrieben.

Die Einstellung des Farbortes einer Mischung aus Farbstoffen wird im Rahmen der Farbmetrik beschrieben. Hierzu werden die Spektren der Einzelfarbstoffe unter Berücksichtigung des Lambert-Beer'schen Gesetzes zu einem Gesamtspektrum berechnet und unter der zugehörigen Beleuchtung, z.B. für Tageslicht die Lichtart D65, gemäß den Regeln der Farbmetrik in die entsprechenden Farborte und Helligkeitswerte umgerechnet. Die Lage des Weißpunktes ist durch die jeweilige Lichtart, z.B. D65 festgelegt und in Tabellen (z. b. obenstehende Literaturstelle) aufgeführt. Durch Änderung der Anteile der verschiedenen Farbstoffe lassen sich verschiedene Farborte einstellen.

Gemäß einer bevorzugten Ausführungsform enthält die Schaltschicht zusätzlich zu der mindestens einen Verbindung der Formel (I) einen oder mehrere dichroitische Farbstoffe, die Licht im roten und NIR-Bereich, d.h. von 600 bis 2000 nm Wellenlänge absorbieren, bevorzugt im Bereich von 650 bis 1800 nm, besonders bevorzugt im Bereich von 650 bis 1300 nm. In einer bevorzugten Ausführung sind diese dichroitischen Farbstoffe gewählt aus Azoverbindungen, Anthrachinonen, Methinverbindungen, Azomethinverbindungen, Merocyaninverbindungen, Naphthochinonen, Tetrazinen, Perylenen, Terrylenen, Quaterrylenen, höheren Rylenen, Pyrromethenen, Azofarbstoffen, Nickeldithiolenen, (Metall-)Phthalocyaninen, (Metall-) Naphthalocyaninen, (Metall-)Porphyrinen, Diketopyrrolopyrrolen und Benzothiadiazolen. Besonders bevorzugt sind darunter Perylene und Terrylene.

Der Anteil aller dichroitischen Farbstoffe in der Mischung der Schaltschicht beträgt bevorzugt insgesamt 0.01 bis 10 Gew.-%, besonders bevorzugt 0.1 bis 7 Gew.-% und ganz besonders bevorzugt 0.2 bis 7 Gew.-%.

Die zusätzlichen, von Formel (I) unterschiedlichen, dichroitischen Farbstoffe der Schaltschicht sind bevorzugt ausgewählt aus den in B. Bahadur, Liquid Crystals - Applications and Uses, Vol. 3, 1992, World Scientific Publishing, Abschnitt 11.2.1 angegebenen Farbstoffklassen, und besonders bevorzugt aus den in der darin enthaltenen Tabelle aufgeführten expliziten Verbindungen.

Die genannten von Formel (I) unterschiedlichen Farbstoffe gehören zu den dem Fachmann bekannten Klassen von dichroitischen Farbstoffen, die vielfach in der Literatur beschrieben sind. So sind z.B. Anthrachinonfarbstoffe beschrieben in EP 34832, EP 44893, EP 48583, EP 54217, EP 56492, EP 59036, GB 2065158, GB 2065695, GB 2081736, GB 2082196, GB 2094822, GB 2094825, JP-OS 55-123673, DE 3017877, DE 3040102, DE 3115147, DE 3115762, DE 3150803 und DE 3201120, Naphthochinonfarbstoffe beschrieben in DE 3126108 und DE 3202761, Azofarbstoffe in EP 43904, DE 3123519, WO 82/2054, GB 2079770, JP-OS 56-57850, JP-OS 56-104984, US 4308161, US 4308162, US 4340973, T. Uchida, C. Shishido, H. Seki und M. Wada: Mol. Cryst. Lig. Cryst. 39, 39-52 (1977) und H. Seki, C. Shishido, S. Yasui und T. Uchida: Jpn. J. Appl. Phys. 21, 191-192 (1982), und Perylene beschrieben in EP 60895, EP 68427 und WO 82/1191. Rylen-Farbstoffe sind beispielsweise beschrieben in EP 2166040, US 2011/0042651, EP 68427, EP 47027, EP 60895, DE 3110960 und EP 698649. Benzothiadiazole sind beispielsweise beschrieben in WO 2014/187529, und Diketopyrrolopyrrole sind beispielsweise beschrieben in WO 2015/090497.

Gemäß einer bevorzugten Ausführungsform enthält die Schaltschicht der Vorrichtung neben Verbindungen der Formel (I) ausschließlich dichroitische Farbstoffe, ausgewählt aus Rylen-Farbstoffen.

Beispiele für bevorzugte weitere von Formel (I) unterschiedlichen dichroitische Farbstoffe, die in der Schaltschicht der Vorrichtung enthalten sein können, sind in der folgenden Tabelle abgebildet:

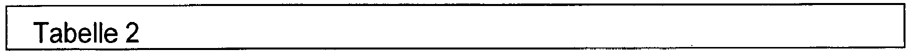

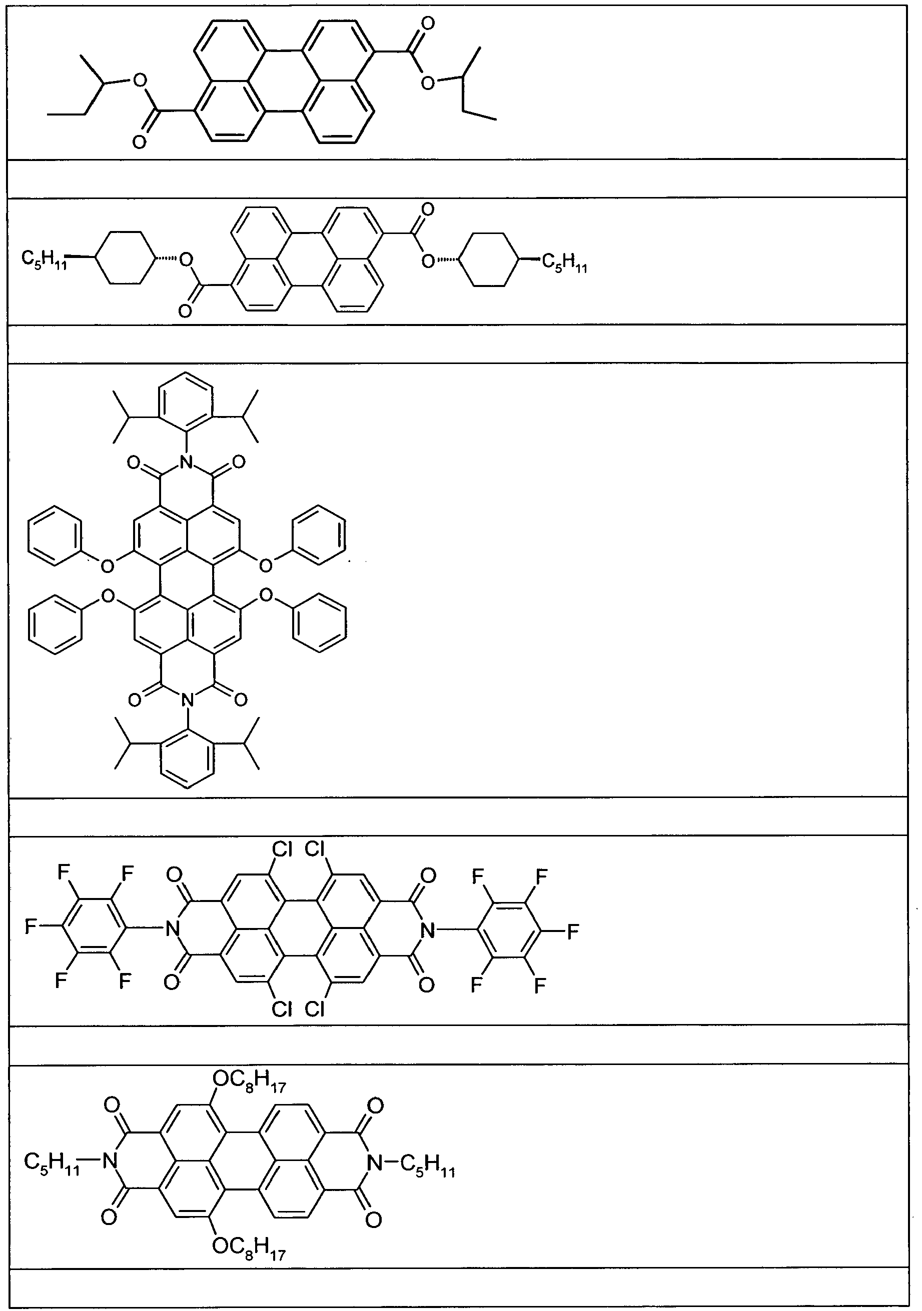

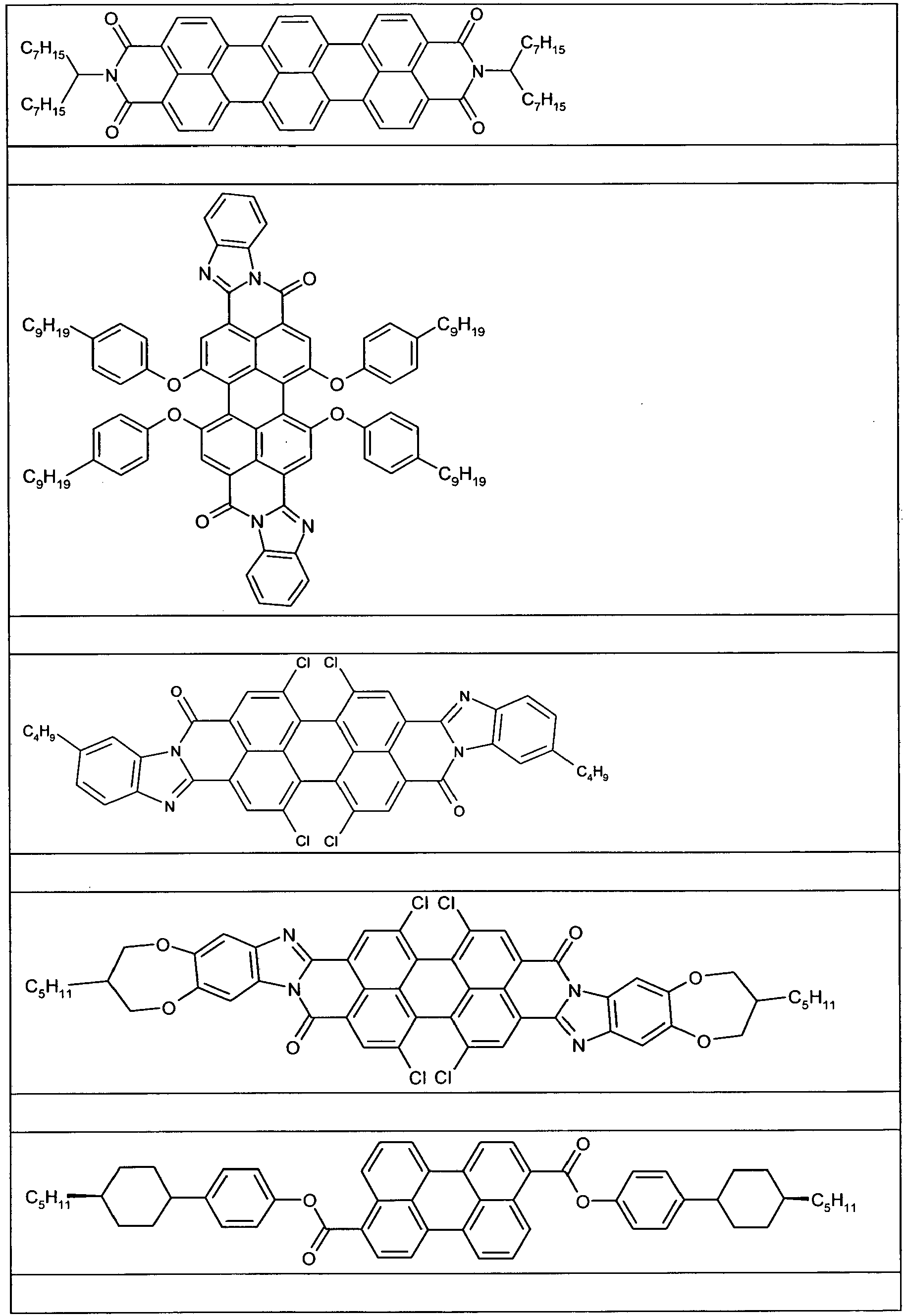

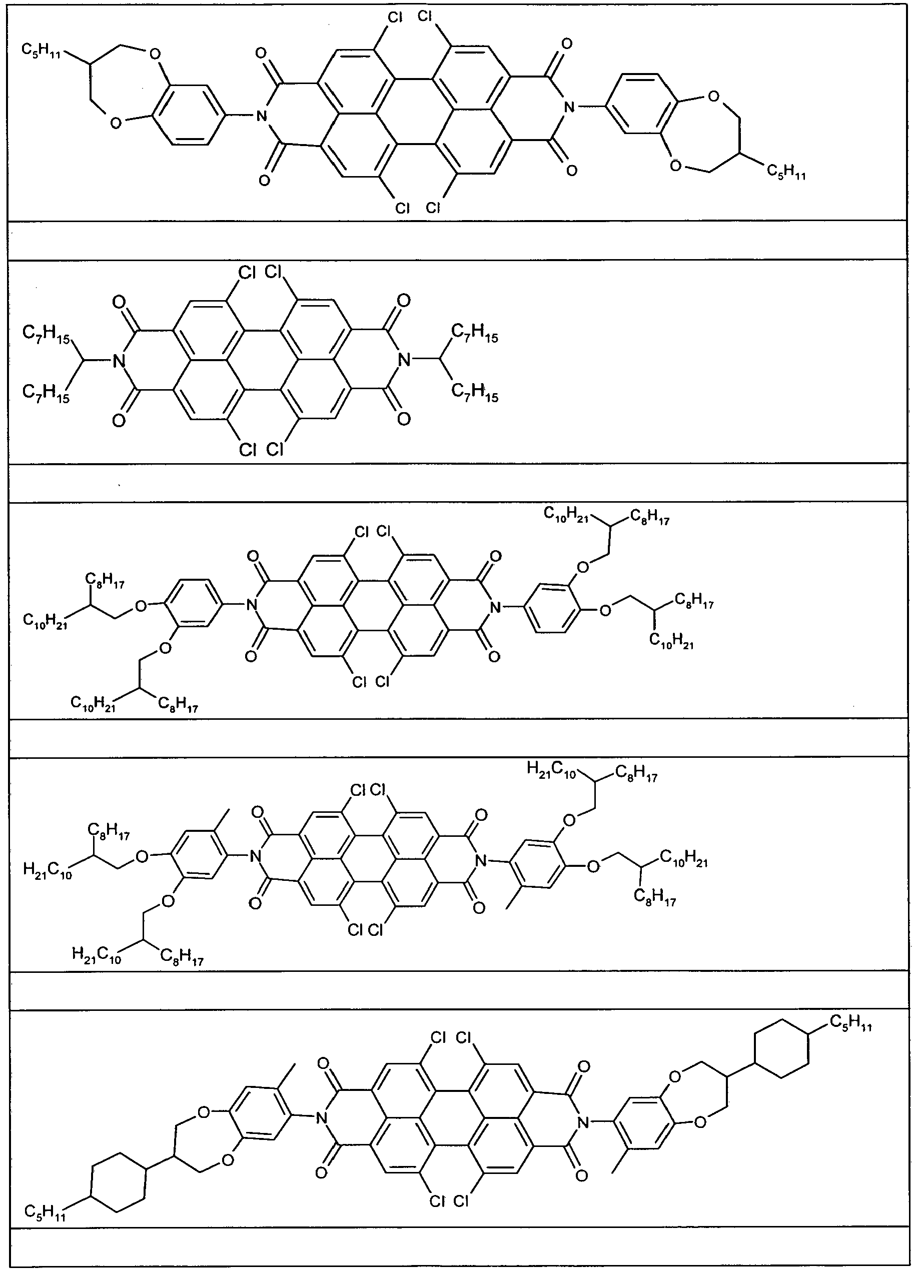

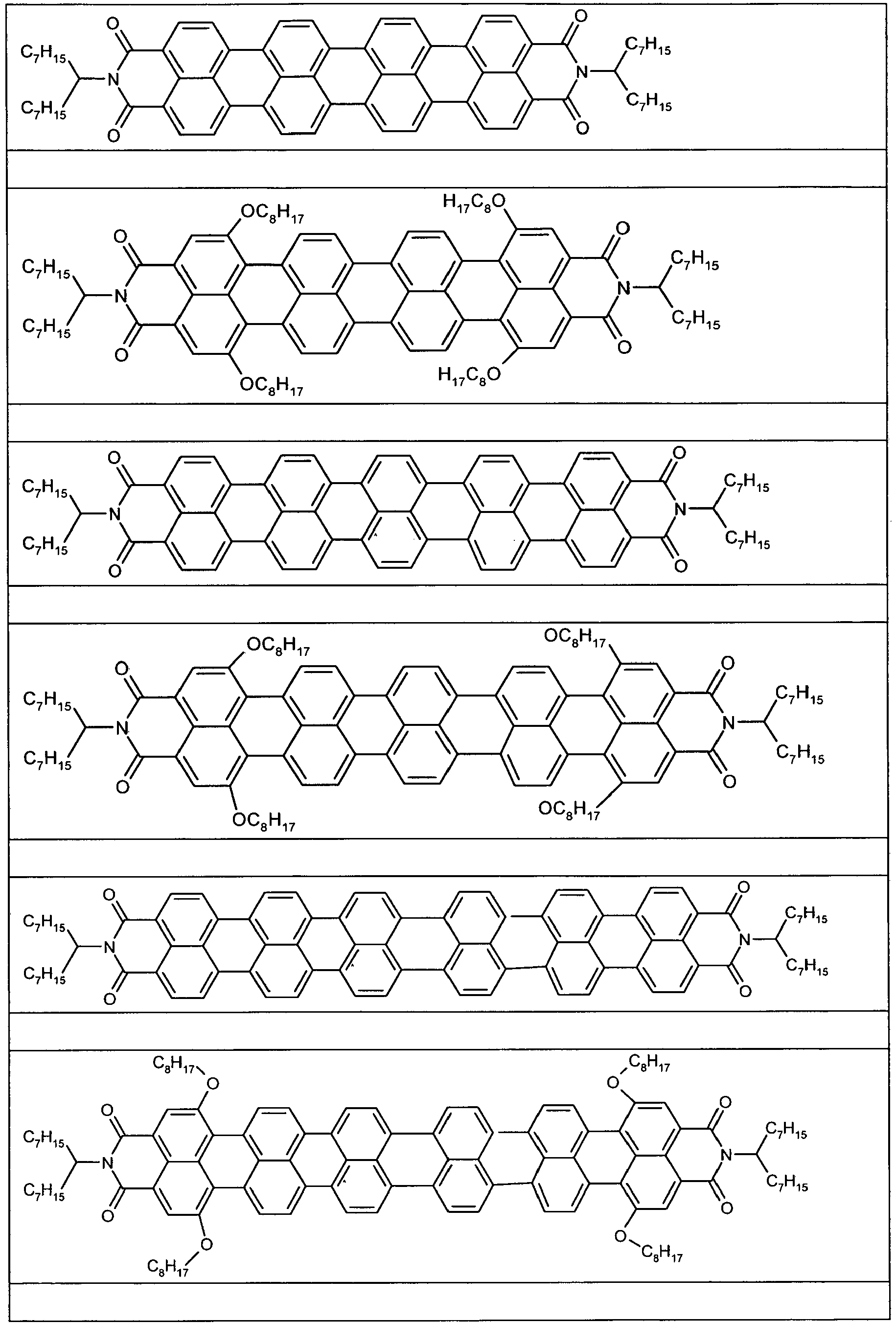

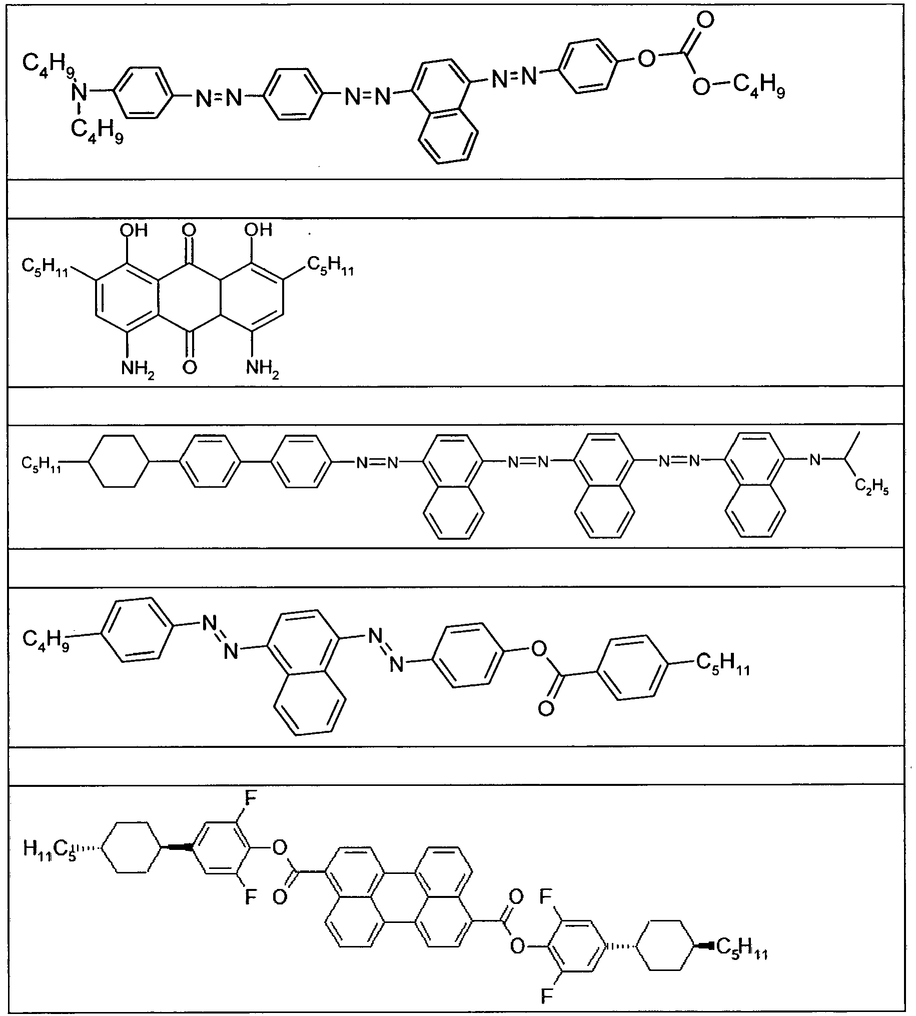

In einer bevorzugten Ausführungsform enthält die Schaltschicht der Vorrichtung eine oder mehrere Quencherverbindungen. Dies ist insbesondere dann bevorzugt, wenn die Vorrichtung in ihrer Schaltschicht einen oder mehrere fluoreszierende Farbstoffe enthält.

Quencherverbindungen sind Verbindungen, welche die Fluoreszenz löschen. Die Quencherverbindungen können in der Schaltschicht die elektronische Anregungsenergie von Nachbarmolekülen, wie z.B. fluoreszierenden Farbstoffen, übernehmen und gehen dabei in einen elektronisch angeregten Zustand über. Der gequenchte fluoreszierende Farbstoff wechselt dadurch in den elektronischen Grundzustand und wird so an der Fluoreszenz oder einer Folgereaktion gehindert. Die Quencherverbindung selbst kehrt durch strahlungslose Desaktivierung oder durch Abgabe von Licht in den Grundzustand zurück und steht zum erneuten Quenchen wieder zur Verfügung.

In der Schaltschicht der Vorrichtung können der Quencherverbindung unterschiedliche Funktionen zukommen. Zum einen kann die Quencherverbindung zur Verlängerung der Lebensdauer eines Farbstoffsystems, durch Deaktivierung elektronischer Anregungsenergie, beitragen. Zum anderen eliminiert die Quencherverbindung ästhetisch ggfs. unerwünschte zusätzliche Farbeffekte, z.B. farbiges Abstrahlen in den Innenraum, die von den fluoreszierenden Farbstoffen in der Schaltschicht ausgehen.

Um ein effektives Quenchen zu erzielen, ist die Quencherverbindung dem jeweiligen Farbstoffsystem, insbesondere dem längstwelligst absorbierenden Farbstoff einer Farbstoffkombination, anzupassen. Wie dies zu tun ist, ist dem Fachmann bekannt.

Bevorzugte Quencherverbindungen sind beispielweise in Tabelle 8.1 auf Seite 279 in Joseph R. Lakowicz, Principles of Fluorescence Spectroscopy, 3rd Edition, 2010, ISBN 10: 0-387-31278-1, Verlag Springer Science+Business Media LLC, beschrieben. Weitere Molekülklassen sind beispielsweise unter den Stichworten Dark-Quencher oder Black Hole Quencher dem Fachmann geläufig. Beispiele sind Azofarbstoffe und Aminoanthrachinone. In der Schaltschicht der Vorrichtung können als Quencherverbindungen auch nicht-fluoreszierende oder nur im NIR fluoreszierende Farbstoffe zum Einsatz kommen.

In einer bevorzugten Ausführungsform der erfindungsgemäßen Schaltschicht sind gegebenenfalls enthaltene Quencherverbindungen so gewählt, dass die Fluoreszenz im sichtbaren Teil des Spektrums unterdrückt wird.

Die Vorrichtung ist bevorzugt zur Regulierung des Energie-Durchtritts in Form von von der Sonne ausgehendem Licht von der Umgebung in einen Innenraum geeignet. Dabei erfolgt der zu regulierende Energie-Durchtritt von der Umwelt (dem Außenraum) in einen Innenraum. Der Innenraum kann dabei ein beliebiger weitgehend von der Umgebung abgeschlossener Raum sein, beispielsweise ein Gebäude, ein Fahrzeug, oder ein Container.

Gegenstand der Erfindung ist daher weiterhin die Verwendung der Vorrichtung zur Regulierung des Energie-Durchtritts von einem Außenraum in einen Innenraum.

Die Vorrichtung kann jedoch auch zur ästhetischen Raumgestaltung eingesetzt werden, beispielsweise für Licht- und Farbeffekte. Beispielsweise können Tür- und Wandelemente enthaltend die erfindungsgemäße Vorrichtung in grau oder in Farbe nach transparent geschaltet werden. Des Weiteren kann die Vorrichtung auch eine weiße oder farbige flächige Hinterleuchtung, die in der Helligkeit moduliert wird, oder eine gelbe flächige Hinterleuchtung, die mit einer blauen Gast-Wirtsanzeige in ihrer Farbe moduliert wird, umfassen. Es können eine oder beide Glasseiten der Vorrichtung mit angerautem oder strukturiertem Glas versehen sein zur Lichtauskopplung und/oder zur Lichteffekterzeugung.

In einer nochmals alternativen Verwendung wird die Vorrichtung zur Regulierung des Lichteinfalls auf die Augen eingesetzt, beispielsweise in Schutzbrillen, Visieren oder Sonnenbrillen, wobei die Vorrichtung in einem Schaltzustand den Lichteinfall auf die Augen gering hält, und in einem anderen Schaltzustand den Lichteinfall weniger stark verringert.

Die Vorrichtung ist bevorzugt in einer Öffnung einer größeren flächigen Struktur angeordnet, wobei die flächige Struktur selbst nicht oder nur geringfügig Energie-Durchtritt zulässt, und wobei die Öffnung relativ gesehen energiedurchlässiger ist. Bevorzugt ist die flächige Struktur eine Wand oder eine sonstige Begrenzung eines Innenraums nach außen. Weiterhin bevorzugt bedeckt die flächige Struktur eine mindestens genauso große Fläche, besonders bevorzugt eine mindestens doppelt so große Fläche wie die Öffnung in ihr, in der die Vorrichtung angeordnet ist.

Bevorzugt ist die Vorrichtung dadurch gekennzeichnet, dass sie eine Flächenausdehnung von mindestens 0.05 m² aufweist, bevorzugt mindestens 0.1 m², besonders bevorzugt mindestens 0.5 m² und ganz besonders bevorzugt mindestens 0.8 m².

Die Vorrichtung ist bevorzugt in einer wie oben beschriebenen, relativ gesehen energiedurchlässigeren Öffnung eines Gebäudes, eines Containers, eines Fahrzeugs oder eines sonstigen weitgehend geschlossenen Raums aufgebracht. Die Vorrichtung kann allgemein für beliebige Innenräume verwendet werden, besonders wenn diese nur begrenzten Luftaustausch mit der Umgebung aufweisen und lichtdurchlässige Begrenzungsflächen aufweisen, durch die Energie-Eintrag von außen in Form von Lichtenergie stattfinden kann. Besonders relevant ist die Verwendung der Vorrichtung für Innenräume, welche starker Sonneneinstrahlung durch lichtdurchlässige Flächen, beispielsweise durch Fensterflächen, ausgesetzt sind.

Die Vorrichtung ist schaltbar. Unter Schaltung wird dabei eine Änderung des Energie-Durchtritts durch die Vorrichtung verstanden. Die Vorrichtung ist bevorzugt elektrisch schaltbar, wie beispielsweise in WO 2009/141295 und in WO 2014/090373 beschrieben wird.

Sie kann aber auch thermisch schaltbar sein, wie beispielsweise in WO 2010/118422 beschrieben. In diesem Fall erfolgt das Schalten bevorzugt durch einen Übergang von einem nematischen zu einem isotropen Zustand durch Änderung der Temperatur der Schaltschicht enthaltend die Verbindung der Formel (I) und ein flüssigkristallines Medium. Im nematischen Zustand liegen die Moleküle des flüssigkristallinen Mediums geordnet vor und damit auch die Verbindung der Formel (I), beispielsweise ausgerichtet parallel zur Oberfläche der Vorrichtung durch die Wirkung einer Orientierungsschicht. Im isotropen Zustand liegen die Moleküle ungeordnet vor, und damit auch die Verbindung der Formel (I). Der Unterschied zwischen geordnetem und ungeordnetem Vorliegen der dichroitischen Verbindung der Formel (I) bewirkt einen Unterschied in der Lichtdurchlässigkeit der Schaltschicht der Vorrichtung, gemäß dem Prinzip, dass dichroitische Verbindungen je nach Orientierung in Bezug auf die Schwingungsebene des Lichts einen höheren oder einen niedrigeren Absorptionskoeffizienten aufweisen.

Ist die Vorrichtung elektrisch schaltbar, umfasst sie bevorzugt zwei oder mehr Elektroden, die zu beiden Seiten der Schaltschicht angebracht sind. Die Elektroden bestehen bevorzugt aus ITO oder aus einer dünnen, bevorzugt transparenten Metall- und/oder Metalloxidschicht, beispielsweise aus Silber oder FTO (Fluor-dotiertes Zinnoxid) oder einem alternativen, dem Fachmann für diese Verwendung bekannten Material. Die Elektroden sind bevorzugt mit elektrischen Anschlüssen versehen. Die Spannung wird bevorzugt durch eine Batterie, einen Akkumulator, oder durch externe Stromversorgung bereitgestellt.

Der Schaltvorgang erfolgt im Fall von elektrischer Schaltung durch eine Ausrichtung der Moleküle des flüssigkristallinen Mediums durch das Anlegen von Spannung.

In einer bevorzugten Ausführungsform wird dabei die Vorrichtung von einem Zustand mit hoher Absorption, d. h. geringer Lichtdurchlässigkeit, der ohne Spannung vorliegt, in einen Zustand mit geringerer Absorption, d. h. höherer Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums, und damit die Moleküle der Verbindung der Formel (I), parallel zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums, und damit die Moleküle der Verbindung der Formel (I), senkrecht zur Ebene der Schaltschicht vorliegen.

In einer zur oben genannten Ausführungsform alternativen Ausführungsform wird die Vorrichtung von einem Zustand mit geringer Absorption, d. h. hoher Lichtdurchlässigkeit, der ohne Spannung vorliegt, in einen Zustand mit höherer Absorption, d. h. geringerer Lichtdurchlässigkeit, überführt. Bevorzugt ist das flüssigkristalline Medium der Schaltschicht in beiden Zuständen nematisch. Der spannungslose Zustand ist bevorzugt dadurch gekennzeichnet, dass die Moleküle des flüssigkristallinen Mediums der Schaltschicht, und damit die Moleküle der Verbindung der Formel (I), senkrecht zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle des flüssigkristallinen Mediums der Schaltschicht, und damit die Moleküle der Verbindung der Formel (I), parallel zur Ebene der Schaltschicht vorliegen.

Gemäß einer bevorzugten Ausführungsform der Erfindung kann die Vorrichtung ohne externe Stromversorgung betrieben werden, indem die nötige Energie durch eine Solarzelle oder eine sonstige Vorrichtung zur Umwandlung von Licht und/oder Wärmeenergie in elektrische Energie bereitgestellt wird, die mit der Vorrichtung verbunden ist. Die Bereitstellung der Energie durch die Solarzelle kann direkt oder indirekt, d. h. über eine dazwischengeschaltete Batterie oder Akkumulator oder sonstige Einheit zur Speicherung von Energie, erfolgen. Bevorzugt ist die Solarzelle außen an der Vorrichtung angebracht oder sie ist innerer Bestandteil der Vorrichtung, wie beispielsweise in WO 2009/141295 offenbart. Bevorzugt sind dabei insbesondere Solarzellen, welche bei diffusem Licht besonders effizient sind, sowie transparente Solarzellen.

Die Vorrichtung weist bevorzugt die folgende Schichtenabfolge auf, wobei zusätzlich weitere Schichten vorhanden sein können. Bevorzugt grenzen die im Folgenden angegebenen Schichten in der Vorrichtung direkt aneinander.
- Substratschicht, bevorzugt aus Glas oder Polymer
- elektrisch leitfähige transparente Schicht, bevorzugt aus ITO
- Orientierungsschicht
- Schaltschicht, enthaltend eine oder mehrere Verbindungen der Formel (I)
- Orientierungsschicht
- elektrisch leitfähige transparente Schicht, bevorzugt aus ITO
- Substratschicht, bevorzugt aus Glas oder Polymer

Die bevorzugten Ausführungsformen der einzelnen Schichten werden im Folgenden dargestellt.

Bevorzugt umfasst die Vorrichtung eine oder mehrere, besonders bevorzugt zwei Orientierungsschichten. Die Orientierungsschichten liegen bevorzugt direkt angrenzend an die beiden Seiten der Schaltschicht enthaltend die Verbindung der Formel (I) vor.

Als Orientierungsschichten der Vorrichtung können beliebige dem Fachmann hierzu bekannte Schichten verwendet werden. Bevorzugt sind Polyimid-Schichten, besonders bevorzugt Schichten aus geriebenem Polyimid. Auf bestimmte, dem Fachmann bekannte Art geriebenes Polyimid führt zu einer Ausrichtung der Moleküle des flüssigkristallinen Mediums in Reiberichtung, wenn die Moleküle parallel zur Orientierungsschicht vorliegen (planare Ausrichtung). Dabei ist es bevorzugt, dass die Moleküle des flüssigkristallinen Mediums nicht völlig planar auf der Orientierungsschicht vorliegen, sondern einen leichten Aufstellwinkel aufweisen (pretilt). Zum Erreichen von vertikaler Ausrichtung der Verbindungen des flüssigkristallinen Mediums zur Oberfläche der Orientierungsschicht (homeotrope Ausrichtung) wird bevorzugt auf bestimmte Art behandeltes Polyimid als Material für die Orientierungsschicht eingesetzt (Polyimid für sehr hohe Pretiltwinkel). Weiterhin können Polymere, die durch einen Belichtungsvorgang mit polarisiertem Licht erhalten wurden, als Orientierungsschicht zum Erreichen einer Ausrichtung der Verbindungen des flüssigkristallinen Mediums gemäß einer Orientierungsachse verwendet werden (photoalignment).

Weiterhin bevorzugt ist in der Vorrichtung die Schaltschicht zwischen zwei Substratschichten angeordnet bzw. von diesen eingeschlossen. Die Substratschichten können beispielsweise aus Glas oder einem Polymer, bevorzugt einem lichtdurchlässigen Polymer, bestehen.

Bevorzugt ist die Vorrichtung dadurch gekennzeichnet, dass sie keinen Polarisator auf Polymerbasis, besonders bevorzugt keinen in fester Materiephase vorliegenden Polarisator und ganz besonders bevorzugt überhaupt keinen Polarisator umfasst.

Die Vorrichtung kann jedoch gemäß einer alternativen Ausführungsform auch einen oder mehrere Polarisatoren aufweisen. Bevorzugt sind die Polarisatoren in diesem Fall Linearpolarisatoren.

Wenn genau ein Polarisator vorhanden ist, so ist dessen Absorptionsrichtung bevorzugt senkrecht stehend zur Orientierungsachse der Verbindungen des flüssigkristallinen Mediums der Vorrichtung auf derjenigen Seite der Schaltschicht, auf der sich der Polarisator befindet.

In der Vorrichtung können sowohl absorptive als auch reflektive Polarisatoren eingesetzt werden. Bevorzugt werden Polarisatoren verwendet, die als dünne optische Filme vorliegen. Beispiele für reflektive Polarisatoren, die in der Vorrichtung verwendet werden können, sind DRPF- (diffusive reflective polariser film, 3M), DBEF- (dual brightness enhanced film, 3M), DBR- (layered-polymer distributed Bragg reflectors, wie in US 7,038,745 und US 6,099,758 beschrieben) und APF-Filme (advanced polariser film, 3M, vgl. Technical Digest SID 2006, 45.1, US 2011/0043732 und US 7,023,602). Weiterhin können Polarisatoren basierend auf Drahtgittern (WGP, wire-grid-polarisers), welche Infrarotlicht reflektieren, eingesetzt werden. Beispiele für absorptive Polarisatoren, welche in den Vorrichtungen eingesetzt werden können, sind der Itos XP38-Polarisatorfilm und der Nitto Denko GU-1220DUN-Polarisatorfilm. Ein Beispiel für einen circularen Polarisator, welcher erfindungsgemäß verwendet werden kann, ist der Polarisator APNCP37-035-STD (American Polarizers). Ein weiteres Beispiel ist der Polarisator CP42 (ITOS).

Weiterhin bevorzugt enthält die Vorrichtung ein Lichtleitsystem, das Licht zu einer Solarzelle oder einer sonstigen Vorrichtung zur Umwandlung von Licht und/oder Wärmeenergie in elektrische Energie leitet, bevorzugt wie in WO 2009/141295 beschrieben. Das Lichtleitsystem sammelt und konzentriert Licht, das auf die Vorrichtung trifft. Bevorzugt sammelt und konzentriert es Licht, das von fluoreszierenden dichroitischen Farbstoffen in der Schaltschicht emittiert wird. Das Lichtleitsystem steht mit einer Vorrichtung zur Umwandlung von Lichtenergie in elektrische Energie, bevorzugt einer Solarzelle, in Kontakt, so dass das gesammelte Licht konzentriert auf diese trifft. In einer bevorzugten Ausführungsform der Erfindung ist die Vorrichtung zur Umwandlung von Lichtenergie in elektrische Energie am Rand der Vorrichtung angebracht, in diese integriert und elektrisch mit Mitteln zur elektrischen Schaltung der Vorrichtung verbunden.

In einer bevorzugten Ausführungsform ist die Vorrichtung Bestandteil eines Fensters, besonders bevorzugt eines Fensters enthaltend mindestens eine Glasfläche, ganz besonders bevorzugt eines Fensters, welche Mehrscheiben-Isolierglas enthält.

Unter Fenster wird dabei insbesondere eine Struktur in einem Gebäude verstanden, welche einen Rahmen und mindestens eine von diesem Rahmen umfasste Glasscheibe enthält. Bevorzugt enthält sie einen wärmeisolierenden Rahmen und zwei oder mehr Glasscheiben (Mehrscheiben-Isolierglas).

Gemäß einer bevorzugten Ausführungsform ist die Vorrichtung direkt auf einer Glasfläche eines Fensters aufgebracht, besonders bevorzugt im Zwischenraum zwischen zwei Glasscheiben von Mehrscheiben-Isolierglas.

Ein Fenster enthaltend eine erfindungsgemäße Vorrichtung, bevorzugt mit den oben angegebenen bevorzugten Merkmalen, ist weiterer Gegenstand der Erfindung.

### Abbildung

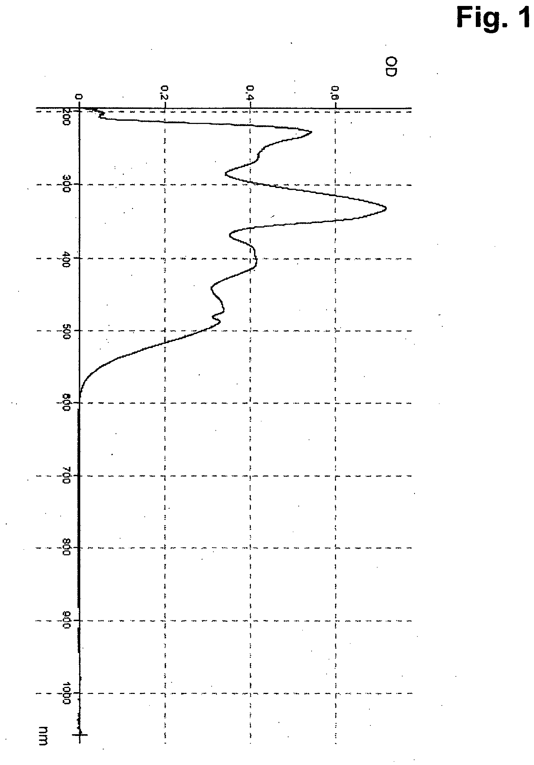

Die Abbildung 1 zeigt ein UV-VIS Spektrum der gemäß Ausführungsbeispiel 2 hergestellten Verbindung V2 mit Absorptionspeaks bei 227, 332, 404, 471 und 487 Nanometern. Gezeigt ist die Absorption (OD) im Verhältnis zur Wellenlänge (nm).

### Ausführungsbeispiele

Die Beispiele zeigen die Herstellung dichroitischer Fluoreszenzfarbstoffe auf Basis von 2-(2,5,7-Trithia-1,3-diaza-s-indacen-6-yliden)malononitril (TDIM). Sie sollen die vorliegende Erfindung verdeutlichen und sind nicht beschränkend auszulegen.

### Beispiel 1a: Herstellung der Verbindung V1

Gemäß dem in Schema 4 gezeigten Syntheseweg wird Verbindung **3** aus Verbindung **1** hergestellt:

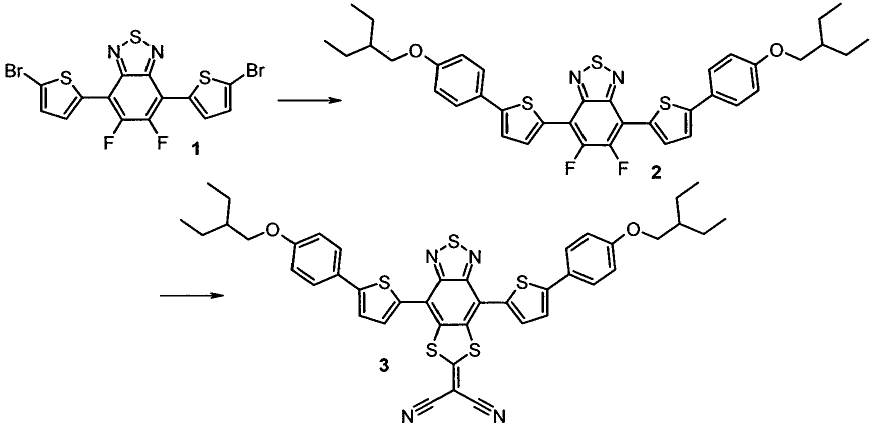

### Reaktionsschema 4

Herstellung der Verbindung **2**: Eine Mischung von **1** (Schroeder et al., 2012; Li et al., 2014) (1.50 g, 3.04 mmol), (2-Ethylbutoxy)benzolboronsäure (1.62 g, 7.3 mmol), Tris(dibenzylidenaceton)dipalladium(0) (32 mg, 0.035 mmol), Tris(o-tolyl)phosphin (42 mg, 0.138 mmol), Toluol (52 mL) und 2 M wäßrige Na₂CO₃-Lösung (30 mL) werden unter Argonatmosphäre 18 h zum Rückfluß erhitzt. Man arbeitet wie üblich wäßrig auf. Das aus den organischen Extrakten gewonnene Rohprodukt wird über eine Kieselgelfritte filtriert (Toluol/n-Heptan 1:1) und anschließend drei Mal aus Toluol kristallisiert. Ausbeute der Verbindung **2:** (1.3 g, 62%) als rote Kristalle, HPLC-Reinheit 99.5%.

Herstellung der Verbindung **3**: Eine Mischung von **2** (1.00 g, 1.45 mmol) und Dimercaptomethylenmalononitril Dinatriumsalz (Hatchard, 1964) (350 mg, 1.88 mmol) und NMP (50 mL) werden unter Argonatmosphäre 18 h bei 90°C gerührt. Man gießt in Wasser (300 mL) und läßt 18 h auskristallisieren. Der ausgefallene Feststoff wird abgesaugt und über eine Kieselgelfritte (Toluol/*n*-Heptan 1:1) filtriert. Den Rückstand kristallisiert man aus heißem Toluol/*n*-Heptan 5:3 um. Ausbeute der Verbindung **3:** (370 mg, 32%) als rote Kristalle, HPLC-Reinheit 99.7%. HR-MS (C₄₂H₃₉O₂N₄S₅): 791.16664.

### Beispiel 1b: Herstellung der Verbindung V2

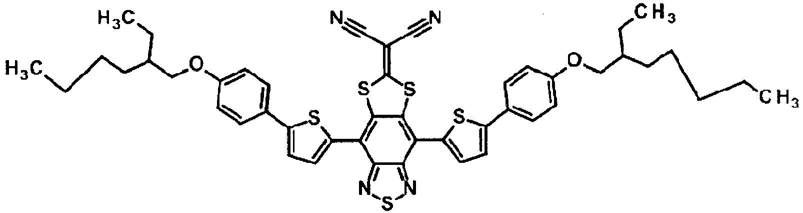

Auf analogem Weg zu Beispiel 1a wird die Verbindung V2 hergestellt, durch Kupplung einer mit 2-Ethylheptyloxy substituierten Phenylboronsäure anstelle der in Beispiel 1a angegebenen 2-Ethylbutoxyphenylboronsäure.

### Beispiel 2: Herstellung der Verbindung V3

Gemäß dem in Schema 5 gezeigten Syntheseweg wird Verbindung **5** aus Verbindung **1** hergestellt:

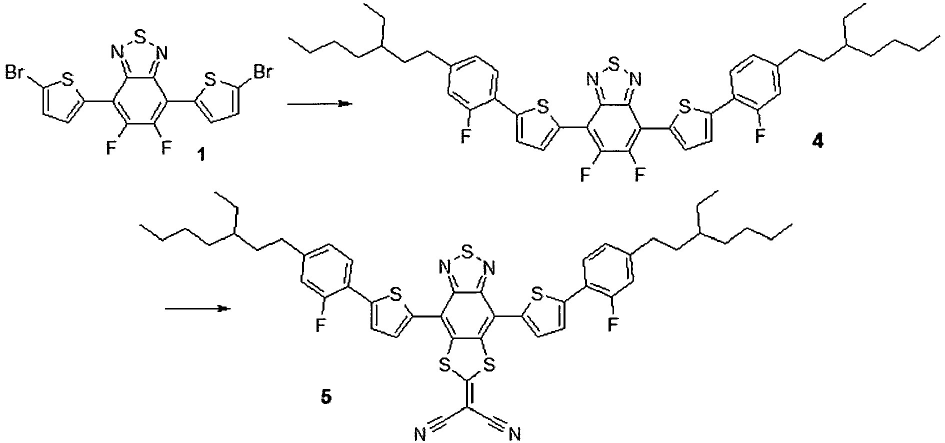

### Reaktionsschema 5

Herstellung der Verbindung **4:** Eine Mischung von Verbindung **1** (Schroeder et al., 2012; Li et al., 2014) (8.00 g, 16.19 mmol), 4-(2-Ethylheptyl)-2-fluorbenzolboronsäure (8.80 g, 33.06 mmol), Tris(dibenzylidenaceton)dipalladium(0) (171 mg, 0.187 mmol), Tris(o-tolyl)phosphin (224 mg, 0.736 mmol), Toluol (280 mL) und 2 M wäßrige Na₂CO₃-Lösung (65 mL) wird unter Argonatmosphäre 18 h zum Rückfluß erhitzt. Man arbeitet wie üblich wäßrig auf. Das aus den organischen Extrakten gewonnene Rohprodukt wird über eine Kieselgelfritte filtriert (Toluol/*n*-Heptan 1:4) und anschließend zweimal aus *n*-Heptan/Toluol 1:1 kristallisiert. Ausbeute der Verbindung **4:** (8.2 g, 66%) als rote Kristalle, HPLC-Reinheit 99.3%.

Herstellung der Verbindung **5**: Eine Mischung von **4** (1.94 g, 2.5 mmol) und Dimercaptomethylenmalononitril Dinatriumsalz (Hatchard, 1964) (930 mg, 5.00 mmol) und NMP (25 mL) wird unter Argonatmosphäre 24 h bei RT gerührt. Man gießt in Wasser (200 mL) und läßt 1 h auskristallisieren. Der ausgefallene Feststoff wird aus Toluol (30 mL) umkristallisiert und anschließend über eine Kieselgelfritte (Toluol) filtriert. Den Rückstand kristallisiert man nochmals aus Toluol (20 mL) um. Ausbeute der Verbindung **5:** (1.1 g, 50%) als rote Kristalle, HPLC-Reinheit 99.4%. HR-MS (C₄₈H₄₉N₄F₂S₅): 879.25234. Abbildung 1 zeigt ein UV-VIS Spektrum der Verbindung **5** (1,3 mg in 100 mL Tetrahydrofuran) mit Absorptionspeaks bei 227, 332, 404, 471 und 487 Nanometern.

### Beispiel 3: Vermessung der UV-VIS-Absorptionsspektren der Verbindungen V1 und V3

Von den beiden oben genannten Verbindungen werden jeweils Absorptionsspektren in THF (Verbindung V1, 1,3 mg in 100 mL Tetrahydrofuran, s. Fig. 1), bzw. in Dichlormethan (Verbindung V3) aufgenommen.

Die Spektren weisen die folgenden Peaks auf:

| Verbindung | Absorptionspeaks bei [nm] |
|---|---|
| V1 | 330; 345; 400; 520 |
| V3 | 227; 332; 404; 471; 487 |

### Beispiel 4: Verwendung von flüssigkristallinen Medien enthaltend die erfindungsgemäßen Farbstoffe in Vorrichtungen zur Regulierung des Licht-Durchtritts

Um die Vorrichtungen herzustellen, wird die Flüssigkristallmischung enthaltend einen der erfindungsgemäßen Farbstoffe in den Zwischenraum der folgenden Schichtenanordnung gefüllt (Einzelzelle):
- Substratschicht
- ITO-Schicht
- Polyimid-Orientierungsschicht
- mit Spacern offengehaltener Zwischenraum
- Polyimid-Orientierungsschicht
- ITO-Schicht
- Substratschicht; bzw. in die Zwischenräume der folgenden Schichtenanordnung gefüllt (Doppelzelle):
   -Substratschicht
   - ITO-Schicht
   - Polyimid-Orientierungsschicht
   - mit Spacern offengehaltener Zwischenraum
   - Polyimid-Orientierungsschicht
   - ITO-Schicht
   -Substratschicht
   -Substratschicht
   - ITO-Schicht
   - Polyimid-Orientierungsschicht
   - mit Spacern offengehaltener Zwischenraum
   - Polyimid-Orientierungsschicht
   - ITO-Schicht
   -Substratschicht,
   also aus zwei hintereinander angeordneten Einzelzellen, wobei die Orienterungsschichten der zweiten Einzelzelle gegenüber den Orientierungsschichten der ersten Einzelzelle eine um 90° gedrehte Reibungsrichtung aufweisen (gekreuzte Einzelzellen).

Die Flüssigkristallschicht ist in dieser Anordnung planar mit antiparallelem Pretiltwinkel orientiert. Diese Orientierung wird durch zwei antiparallel zueinander geriebene Polyimidschichten erreicht. Die Dicke der flüssigkristallinen Schicht wird durch Spacer definiert und beträgt üblicherweise 25 µm.

Es werden Werte für den Lichttransmissionsgrad τᵥ jeweils für den dunklen und den hellen Schaltzustand der Vorrichtung ermittelt und im Folgenden aufgeführt. Der helle Schaltzustand wird durch Anlegen einer Spannung erreicht, während der dunkle Schaltzustand ohne Spannung vorliegt. Weiterhin wird der Farbort der Vorrichtung (in CIE-Koordinaten) im Dunkel- und im Hellzustand ermittelt.

Die Messung erfolgt mit der Vorrichtung enthaltend das flüssigkristalline Medium mit Farbstoffen im Messstrahl und einer baugleichen Vorrichtung, entsprechend ohne die Farbstoffe im Referenzstrahl. Hierdurch werden Reflexions- und Absorptionsverluste der Zelle eliminiert.

Der Wert τᵥ und die CIE-Koordinaten (x,y) sind wie folgt definiert:
τᵥ= Lichttransmissionsgrad, bestimmt nach DIN EN410
Der Farbort (für weiß, grau schwarz) der hier zugrundegelegten Normlichtart D65 liegt bei x=0.3127 und y=0.3290 (Manfred Richter, Einführung in die Farbmetrik, zweite Auflage 1991, ISBN 3-11-008209-8). Die angegebenen Farborte (x,y) beziehen sich alle auf die Normlichtart D65 und den 2° Normalbeobachter nach CIE 1931.

Die folgende Mischung dient als Hostmischung (M1)

| Zusammensetzung der Hostmischung M1 | | |
|---|---|---|
| Klärpunkt | 114.5°C | |
| Delta-n | 0.1342 | |
| nₑ | 1.6293 | |
| nₒ | 1.4951 | |

| Zusammensetzung | Verbindung | |
|---|---|---|
| | CPG-3-F | Gew.-% |
| | CPG-5-F | 5 |
| | CPU-3-F | 5 |
| | CPU-5-F | 15 |
| | CP-3-N | 15 |
| | CP-5-N | 16 |
| | CCGU-3-F | 16 |
| | CGPC-3-3 | 7 |
| | CGPC-5-3 | 4 |
| | CGPC-5-5 | 4 |
| | CCZPC-3-3 | 4 |
| | CCZPC-3-4 | 3 |
| | CCZPC-3-5 | 3 |

Dies sind die Strukturen der weiteren Verbindungen, die im Folgenden verwendet werden:

| |
|---|
| 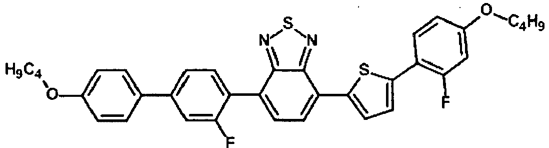 |
| D1 |
| 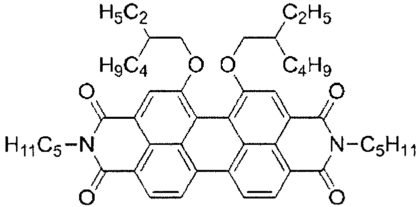 |
| D2 |
| 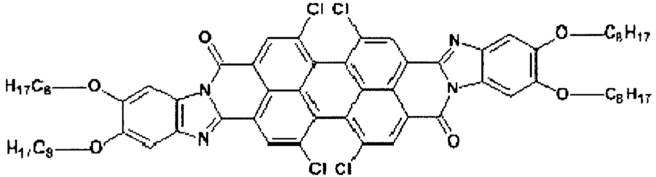 |
| D3 |
| 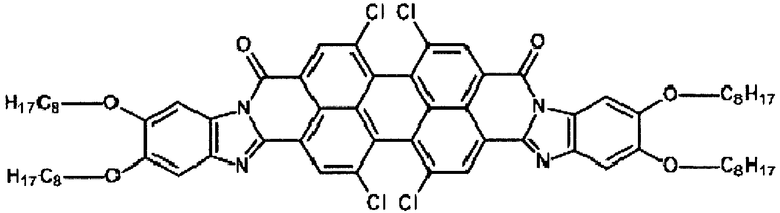 |
| D4 |

### Beispiel 4a

Es wird die folgende flüssigkristalline Mischung verwendet:

| Bestandteil | Anteil [%] |
|---|---|
| M1 | 99.029 |
| D1 | 0.188 |
| V1 | 0.118 |
| D2 | 0.061 |
| D3 | 0.604 |

Erhaltene Messwerte für die Vorrichtung (Doppelzelle, jeweils 25 µm):
- Dunkelzustand: x=0.312; y= 0.329; τᵥ =15%
- Hellzustand: x=0.334; y=0.358; τᵥ =70.6%

### Beispiel 4b

| Bestandteil | Anteil [%] |
|---|---|
| M1 | 98.035 |
| D1 | 0.221 |
| V1 | 0.243 |
| D2 | 0.158 |
| D3 | 0.680 |
| D4 | 0.663 |

Erhaltene Messwerte für die Vorrichtung (Einzelzelle, 25µm):
- Dunkelzustand: x=0.313; y= 0.329; τᵥ =38%
- Hellzustand: x=0.322; y=0.344; τᵥ =72%

### Beispiel 4c

| Bestandteil | Anteil [%] |
|---|---|
| M1 | 99.029 |
| D1 | 0.153 |
| V1 | 0.20 |
| V2 | 0.255 |
| D2 | 0.199 |
| D3 | 1.111 |
| D4 | 1.156 |

Erhaltene Messwerte für die Vorrichtung (Einzelzelle, 25µm):
- Dunkelzustand: x=0.313; y= 0.329; τᵥ =30%
- Hellzustand: x=0.322; y=0.346; τᵥ =60%

Die Beispiele zeigen, dass sich die Vorrichtung durch Anlegen einer Spannung von einem Dunkelzustand mit deutlich geringerer Lichttransmission zu einem Hellzustand mit deutlich erhöhter Lichttransmission schalten lässt.

### Literatur

Hatchard, W. R., 1964, J. Org. Chem. 29, 660-665.
Li, Kang, Gong, Zhang, Li, Li, Dong, Hu, Bo, 2014; J. Mater. Chem. C, 2, 5116-5123.
Schroeder, Huang, Ashraf, Smith, D'Angelo, Watkins, Anthopoulos, Durrant, McCulloch; Adv. Funct. Mater. 2012, 22, 1663-1670.
Zhang, Gorohmaru, Kadowaki, Kobayashi, Ishi-i, Thiemann, Mataka; J. Mater. Chem. 2004, 14, 1901-1904.
Zhang, Cien, Chen, Yip, Sun, Davies, Chen, Jen, Chem. Commun. 2011, 47, 11026-11028.

## Patentansprüche

1. Verbindung der Formel (I): 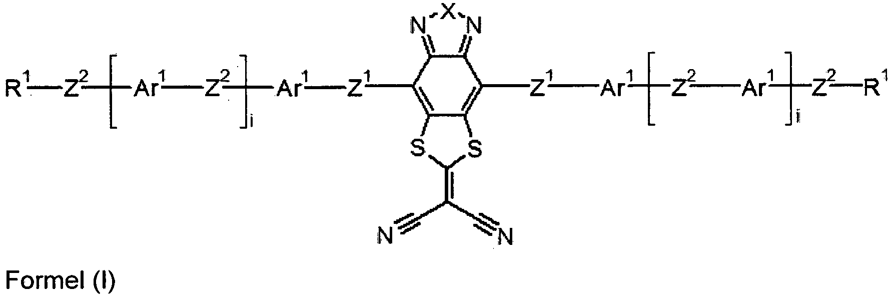 wobei gilt:
X ist gleich S oder Se;
Z¹ ist unabhängig voneinander eine Einfachbindung, -CR³=CR³- oder -C≡C-; oder zwei, drei, vier oder fünf miteinander kombinierte Gruppen, gewählt aus den Gruppen -CR³=CR³- und -C≡C-;
Z² ist unabhängig voneinander eine Einfachbindung, O, S, C(R³)₂, -CR³=CR³- oder -C≡C-; oder zwei, drei, vier oder fünf miteinander kombinierte Gruppen, gewählt aus den Gruppen O, S, C(R³)₂, -CR³=CR³- und -C≡C-;
Ar¹ ist unabhängig voneinander eine Aryl- oder Heteroarylgruppe mit 5 bis 30 aromatischen Ringatomen, die mit einem oder mehreren Resten R⁴ substituiert sein kann;
R¹ ist unabhängig voneinander H, D, F, CN, N(R⁵)₂, oder eine Alkyl-, Alkoxy- oder Thioalkoxygruppe mit 1 bis 20 C-Atomen, die mit einem oder mehreren Resten R⁵ substituiert sein kann, wobei eine oder mehrere CH₂-Gruppen in den Alkyl-, Alkoxy- oder Thioalkoxygruppen durch -R⁵C=CR⁵⁻, -C≡C-, C=O, C=S, -C(=O)O-, -OC(=O)-, Si(R⁵)₂, NR⁵, -O- oder -S- ersetzt sein können;
R³, R⁴ sind unabhängig voneinander H, D, F, Cl, CN, oder eine Alkyl-, Alkoxy-, oder Thioalkoxygruppe mit 1 bis 10 C-Atomen, die mit einem oder mehreren Resten R⁵ substituiert sein kann, wobei eine oder mehrere CH₂-Gruppen in den Alkyl-, Alkoxy- oder Thioalkoxygruppen durch -R⁵C=CR⁵⁻, -C≡C-, C=O, C=S, -C(=O)O-, -OC(=O)-, Si(R⁵)₂, NR⁵, -O- oder -S- ersetzt sein können;
R⁵ ist unabhängig voneinander H, D, F, Cl, CN, N(R⁶)₂, eine Alkyl-, Alkoxy- oder Thioalkoxygruppe mit 1 bis 10 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, C=O, C=S, -C(=O)O-, -O(C=O)-, Si(R⁶)₂, NR⁶, -O- oder -S- ersetzt sein können, oder eine Aryl- oder Heteroarylgruppe mit 5 bis 30 aromatischen Ringatomen, die jeweils mit einem oder mehreren Resten R⁶ substituiert sein kann;
R⁶ ist unabhängig voneinander H, F oder ein aliphatischer organischer Rest mit 1 bis 20 C-Atomen, in dem ein oder mehrere H-Atome durch F ersetzt sein können, oder eine Aryl- oder Heteroarylgruppe mit 5 bis 20 C-Atomen, in der ein oder mehrere H-Atome durch F ersetzt werden können; und
i ist unabhängig voneinander gleich 0, 1, 2, 3, 4 oder 5.

2. Verbindung nach Anspruch 1, **dadurch gekennzeichnet, dass** X gleich S ist und/oder dass Z¹ eine Einfachbindung ist.

3. Verbindung nach einem oder mehreren der vorhergehenden Ansprüche, **dadurch gekennzeichnet, dass** Z² unabhängig voneinander für eine Einfachbindung, -C(R³)₂C(R³)₂-, -CR³=CR³-, -C≡C-, -OC(R³)₂- oder -C(R³)₂O- steht.

4. Verbindung nach einem oder mehreren der vorhergehenden Ansprüche, **dadurch gekennzeichnet, dass** Z¹ und Z² für eine Einfachbindung stehen.

5. Verbindung nach einem oder mehreren der vorhergehenden Ansprüche, **dadurch gekennzeichnet, dass** Ar¹ unabhängig voneinander eine Arylgruppe mit 6 bis 15 C-Atomen oder eine Heteroarylgruppe mit 5 bis 15 C-Atomen darstellt, die mit einem oder mehreren Resten R⁴ substituiert sein kann.

6. Verbindung nach einem oder mehreren der vorhergehenden Ansprüche, **dadurch gekennzeichnet, dass** Ar¹ bei jedem Auftreten gewählt ist aus wahlweise mit Resten R⁴ substituiertem Benzol, Fluoren, Naphthalin, Pyridin, Pyrimidin, Pyrazin, Triazin, Thiophen, Thiophen mit ankondensiertem 1,4-Dioxanring, Benzothiophen, Dibenzothiophen, Benzodithiophen, Cyclopentadithiophen, Thienothiophen, Indenothiophen, Dithienopyrrol, Silolodithiophen, Selenophen, Benzoselenophen, Dibenzoselenophen, Furan, Benzofuran, Dibenzofuran und Chinolin.

7. Verbindung nach einem oder mehreren der vorhergehenden Ansprüche, **dadurch gekennzeichnet, dass** mindestens ein Ar¹ gewählt ist aus einer schwefelhaltigen Heteroarylgruppe, die mit einem oder mehreren Resten R⁴ substituiert sein kann.

8. Verbindung nach einem oder mehreren der vorhergehenden Ansprüche, **dadurch gekennzeichnet, dass** R¹ unabhängig voneinander ausgewählt ist aus H, F, oder einer geradkettigen Alkyl- oder Alkoxylgruppe mit 3 bis 20 C-Atomen, die mit einem oder mehreren Resten R⁵ substituiert sein kann, oder einer verzweigten Alkyl- oder Alkoxygruppe mit 3 bis 20 C-Atomen, die mit einem oder mehreren Resten R⁵ substituiert sein kann, oder einer cyclischen Alkylgruppe mit 6 C-Atomen, die mit einem oder mehreren Resten R⁵ substituiert sein kann, wobei in den Alkyl- und Alkoxygruppen eine oder mehrere CH₂-Gruppen durch -O-, -S- oder -R⁵C=CR⁵- ersetzt sein können, oder einer Siloxanylgruppe mit 1 bis 6 Si-Atomen, die mit einem oder mehreren Resten R⁵ substituiert sein kann.

9. Verbindung nach einem oder mehreren der vorhergehenden Ansprüche, **dadurch gekennzeichnet, dass** R¹ unabhängig voneinander eine verzweigte Alkylgruppe mit 4 bis 15 C-Atomen ist.

10. Verbindung nach einem oder mehreren der vorhergehenden Ansprüche, **dadurch gekennzeichnet, dass** der Index i gleich 1 oder 2 ist.

11. Verbindung nach einem oder mehreren der vorhergehenden Ansprüche, **dadurch gekennzeichnet, dass** der Anisotropiegrad R der Verbindung der Formel (I) größer ist als 0.4.

12. Vorrichtung zur Regulierung des Energie-Durchtritts von einem Außenraum in einen Innenraum, wobei die Vorrichtung eine Schaltschicht enthält, wobei die Schaltschicht eine oder mehrere Verbindungen der Formel (I) enthält.

13. Vorrichtung nach Anspruch 12, **dadurch gekennzeichnet, dass** neben der Verbindung der Formel (I) in der Schaltschicht ein flüssigkristallines Medium enthaltend eine oder mehrere verschiedene Verbindungen vorliegt.

14. Vorrichtung nach Anspruch 12 oder 13, **dadurch gekennzeichnet, dass** sie elektrisch schaltbar ist.

15. Vorrichtung nach einem oder mehreren der Ansprüche 12 bis 14, **dadurch gekennzeichnet, dass** sie mit einer Solarzelle oder einer sonstigen Vorrichtung zur Umwandlung von Licht und/oder Wärmeenergie in elektrische Energie verbunden ist.

## Claims

1. Compound of the formula (I): 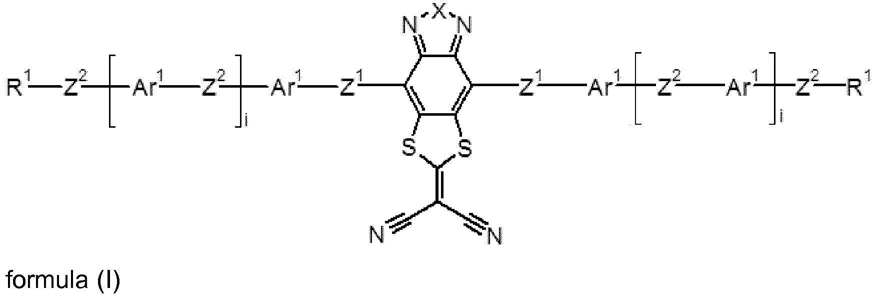 where:
X is equal to S or Se;
Z¹ is, independently of one another, a single bond, -CR³=CR³- or -C≡C-; or two, three, four or five groups combined with one another, selected from the groups -CR³=CR³- and -C≡C-;
Z² is, independently of one another, a single bond, O, S, C(R³)₂, -CR³=CR³- or -C≡C-; or two, three, four or five groups combined with one another, selected from the groups O, S, C(R³)₂, -CR³=CR³- and -C≡C-;
Ar¹ is, independently of one another, an aryl or heteroaryl group having 5 to 30 aromatic ring atoms, which may be substituted by one or more radicals R⁴;
R¹ is, independently of one another, H, D, F, CN, N(R⁵)₂, or an alkyl, alkoxy or thioalkoxy group having 1 to 20 C atoms, which may be substituted by one or more radicals R⁵, where one or more CH₂ groups in the alkyl, alkoxy or thioalkoxy groups may be replaced by -R⁵C=CR⁵-, -C≡C-, C=O, C=S, -C(=O)O-, -OC(=O)-, Si(R⁵)₂, NR⁵, -O- or -S-;
R³, R⁴ are, independently of one another, H, D, F, CI, CN, or an alkyl, alkoxy or thioalkoxy group having 1 to 10 C atoms, which may be substituted by one or more radicals R⁵, where one or more CH₂ groups in the alkyl, alkoxy or thioalkoxy groups may be replaced by -R⁵C=CR⁵-, -C≡C-, C=O, C=S, -C(=O)O-, -OC(=O)-, Si(R⁵)₂, NR⁵, -O- or -S-;
R⁵ is, independently of one another, H, D, F, Cl, CN, N(R⁶)₂, an alkyl, alkoxy or thioalkoxy group having 1 to 10 C atoms or an alkenyl or alkynyl group having 2 to 20 C atoms, where the above-mentioned groups may each be substituted by one or more radicals R⁶ and where one or more CH₂ groups in the above-mentioned groups may be replaced by -R⁶C=CR⁶-, -C≡C-, C=O, C=S, -C(=O)O-, -O(C=O)-, Si(R⁶)₂, NR⁶, -O- or -S-, or an aryl or heteroaryl group having 5 to 30 aromatic ring atoms, which may in each case be substituted by one or more radicals R⁶;
R⁶ is, independently of one another, H, F or an aliphatic organic radical having 1 to 20 C atoms, in which one or more H atoms may be replaced by F, or an aryl or heteroaryl group having 5 to 20 C atoms, in which one or more H atoms may be replaced by F; and
i is, independently of one another, equal to 0, 1, 2, 3, 4 or 5.

2. Compound according to Claim 1, **characterised in that** X is equal to S and/or **in that** Z¹ is a single bond.

3. Compound according to one or more of the preceding claims, **characterised in that** Z² stands, independently of one another, for a single bond, -C(R³)₂C(R³)₂-, -CR³=CR³-, -C≡C-, -OC(R³)₂- or -C(R³)₂O-.

4. Compound according to one or more of the preceding claims, **characterised in that** Z¹ and Z² stand for a single bond.

5. Compound according to one or more of the preceding claims, **characterised in that** Ar¹ represents, independently of one another, an aryl group having 6 to 15 C atoms or a heteroaryl group having 5 to 15 C atoms, which may be substituted by one or more radicals R⁴.

6. Compound according to one or more of the preceding claims, **characterised in that** Ar¹ is selected on each occurrence from benzene, fluorene, naphthalene, pyridine, pyrimidine, pyrazine, triazine, thiophene, thiophene with condensed-on 1,4-dioxane ring, benzothiophene, dibenzothiophene, benzodithiophene, cyclopentadithiophene, thienothiophene, indenothiophene, dithienopyrrole, silolodithiophene, selenophene, benzoselenophene, dibenzoselenophene, furan, benzofuran, dibenzofuran and quinoline, each of which is optionally substituted by radicals R⁴.

7. Compound according to one or more of the preceding claims, **characterised in that** at least one Ar¹ is selected from a sulfur-containing heteroaryl group, which may be substituted by one or more radicals R⁴.

8. Compound according to one or more of the preceding claims, **characterised in that** R¹ is selected, independently of one another, from H, F, or a straight-chain alkyl or alkoxy group having 3 to 20 C atoms, which may be substituted by one or more radicals R⁵, or a branched alkyl or alkoxy group having 3 to 20 C atoms, which may be substituted by one or more radicals R⁵, or a cyclic alkyl group having 6 C atoms, which may be substituted by one or more radicals R⁵, where one or more CH₂ groups in the alkyl and alkoxy groups may be replaced by -O-, -S- or -R⁵C=CR⁵-, or a siloxanyl group having 1 to 6 Si atoms, which may be substituted by one or more radicals R⁵.

9. Compound according to one or more of the preceding claims, **characterised in that** R¹ is, independently of one another, a branched alkyl group having 4 to 15 C atoms.

10. Compound according to one or more of the preceding claims, **characterised in that** the index i is equal to 1 or 2.

11. Compound according to one or more of the preceding claims, **characterised in that** the degree of anisotropy R of the compound of the formula (I) is greater than 0.4.

12. Device for regulating the passage of energy from an outside space into an inside space, where the device contains a switching layer, where the switching layer comprises one or more compounds of the formula (I).

13. Device according to Claim 12, **characterised in that**, besides the compound of the formula (I), a liquid-crystalline medium comprising one or more different compounds is present in the switching layer.

14. Device according to Claim 12 or 13, **characterised in that** it is electrically switchable.

15. Device according to one or more of Claims 12 to 14, **characterised in that** it is connected to a solar cell or another device for conversion of light and/or heat energy into electrical energy.

## Revendications

1. Composé de la formule (I) : 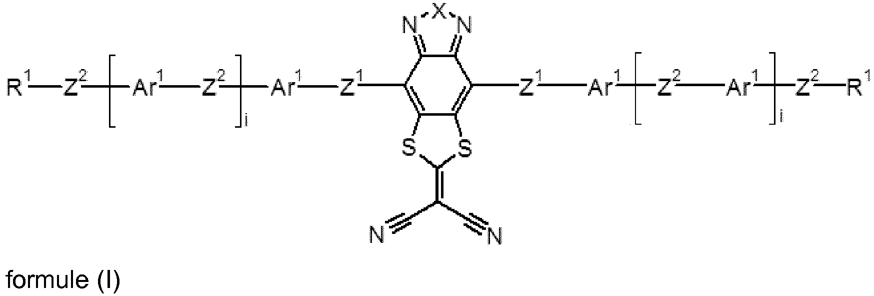 dans laquelle :
X est égal à S ou Se ;
Z¹ est, de manière indépendante les uns des autres, une liaison simple, -CR³=CR³⁻ ou -C≡C- ; ou deux, trois, quatre ou cinq groupes combinés l'un avec l'autre ou les uns avec les autres, sélectionnés parmi les groupes -CR³=CR³⁻ et -C≡C- ;
Z² est, de manière indépendante les uns des autres, une liaison simple, O, S, C(R³)₂, -CR³=CR³⁻ ou -C≡C- ; ou deux, trois, quatre ou cinq groupes combinés l'un avec l'autre ou les uns avec les autres, sélectionnés parmi les groupes O, S, C(R³)₂, -CR³=CR³- et -C≡C- ;
Ar¹ est, de manière indépendante les uns des autres, un groupe aryle ou hétéroaryle qui comporte de 5 à 30 atomes de cycle aromatique, lequel peut être substitué par un radical ou par plusieurs radicaux R⁴;
R¹ est, de manière indépendante les uns des autres, H, D, F, CN, N(R⁵)₂, ou un groupe alkyle, alcoxy ou thioalcoxy qui comporte de 1 à 20 atome(s) de C, lequel peut être substitué par un radical ou par plusieurs radicaux R⁵, où un ou plusieurs groupe(s) CH₂ dans les groupes alkyle, alcoxy ou thioalcoxy peut/peuvent être remplacé(s) par -R⁵C=CR⁵-, -C≡C-, C=O, C=S, -C(=O)O-, -OC(=O)-, Si(R⁵)₂, NR⁵, -O- ou -S- ;
R³, R⁴ sont, de manière indépendante l'un de l'autre, H, D, F, Cl, CN, ou un groupe alkyle, alcoxy ou thioalcoxy qui comporte de 1 à 10 atome(s) de C, lequel peut être substitué par un radical ou par plusieurs radicaux R⁵, où un ou plusieurs groupe(s) CH₂ dans les groupes alkyle, alcoxy ou thioalcoxy peut/peuvent être remplacé(s) par -R⁵C=CR⁵-, -C≡C-, C=O, C=S, -C(=O)O-, -OC(=O)-, Si(R⁵)₂, NR⁵, -O- ou -S- ;
R⁵ est, de manière indépendante les uns des autres, H, D, F, Cl, CN, N(R⁶)₂, un groupe alkyle, alcoxy ou thioalcoxy qui comporte de 1 à 10 atome(s) de C ou un groupe alkényle ou alkynyle qui comporte de 2 à 20 atomes de C, où les groupes qui ont été mentionnés ci-avant peuvent chacun être substitués par un radical ou par plusieurs radicaux R⁶ et où un ou plusieurs groupe(s) CH₂ dans les groupes qui ont été mentionnés ci-avant peut/peuvent être remplacé(s) par -R⁶C=CR⁶-, -C≡C-, C=O, C=S, -C(=O)O-, -O(C=O)-, Si(R⁶)₂, NR⁶, -O- ou -S-, ou un groupe aryle ou hétéroaryle qui comporte de 5 à 30 atomes de cycle aromatique, lequel peut dans chaque cas être substitué par un radical ou par plusieurs radicaux R⁶ ;
R⁶ est, de manière indépendante les uns des autres, H, F ou un radical organique aliphatique qui comporte de 1 à 20 atome(s) de C, où un ou plusieurs atome(s) de H peut/peuvent être remplacé(s) par F, ou un groupe aryle ou hétéroaryle qui comporte de 5 à 20 atomes de C, où un ou plusieurs atome(s) de H peut/peuvent être remplacé(s) par F ; et
i est, de manière indépendante l'un de l'autre, égal à 0, 1, 2, 3, 4 ou 5.

2. Composé selon la revendication 1, **caractérisé en ce que** X est égal à S et/ou **en ce que** Z¹ est une liaison simple.

3. Composé selon une ou plusieurs des revendications qui précèdent, **caractérisé en ce que** Z² représente, de manière indépendante les uns des autres, une liaison simple, -C(R³)₂C(R³)₂-, -CR³=CR³-, -C≡C-, -OC(R³)₂- ou -C(R³)₂O-.

4. Composé selon une ou plusieurs des revendications qui précèdent, **caractérisé en ce que** Z¹ et Z² représentent une liaison simple.

5. Composé selon une ou plusieurs des revendications qui précèdent, **caractérisé en ce que** Ar¹ représente, de manière indépendante les uns des autres, un groupe aryle qui comporte de 6 à 15 atomes de C ou un groupe hétéroaryle qui comporte de 5 à 15 atomes de C, lequel peut être substitué par un radical ou par plusieurs radicaux R⁴.

6. Composé selon une ou plusieurs des revendications qui précèdent, **caractérisé en ce que** Ar¹ est sélectionné pour chaque occurrence parmi benzène, fluorène, naphtalène, pyridine, pyrimidine, pyrazine, triazine, thiophène, thiophène avec un cycle 1,4-dioxane condensé dessus, benzothiophène, dibenzothiophène, benzodithiophène, cyclopentadithiophène, thiénothiophène, indénothiophène, dithiénopyrrole, silolodithiophène, sélénophène, benzosélénophène, dibenzosélénophène, furane, benzofurane, dibenzofurane et quinoline, dont chacun est en option substitué par des radicaux R⁴.

7. Composé selon une ou plusieurs des revendications qui précèdent, **caractérisé en ce qu'**au moins un Ar¹ est sélectionné à partir d'un groupe hétéroaryle contenant du soufre, lequel peut être substitué par un radical ou par plusieurs radicaux R⁴.

8. Composé selon une ou plusieurs des revendications qui précèdent, **caractérisé en ce que** R¹ est sélectionné, de manière indépendante les uns des autres, parmi H, F, ou un groupe alkyle ou alcoxy en chaîne droite qui comporte de 3 à 20 atomes de C, lequel peut être substitué par un radical ou par plusieurs radicaux R⁵, ou un groupe alkyle ou alcoxy ramifié qui comporte de 3 à 20 atomes de C, lequel peut être substitué par un radical ou par plusieurs radicaux R⁵, ou un groupe alkyle cyclique qui comporte 6 atomes de C, lequel peut être substitué par un radical ou par plusieurs radicaux R⁵, où un ou plusieurs groupe(s) CH₂ dans les groupes alkyle et alcoxy peut/peuvent être remplacé(s) par -O-, -S- ou -R⁵C=CR⁵-, ou un groupe siloxanyle qui comporte de 1 à 6 atome(s) de Si, lequel peut être substitué par un radical ou par plusieurs radicaux R⁵.

9. Composé selon une ou plusieurs des revendications qui précèdent, **caractérisé en ce que** R¹ est, de manière indépendante les uns des autres, un groupe alkyle ramifié qui comporte de 4 à 15 atomes de C.

10. Composé selon une ou plusieurs des revendications qui précèdent, **caractérisé en ce que** l'indice i est égal à 1 ou 2.

11. Composé selon une ou plusieurs des revendications qui précèdent, **caractérisé en ce que** le degré d'anisotropie R du composé de la formule (I) est supérieur à 0,4.

12. Dispositif pour réguler le passage de l'énergie depuis un espace extérieur à l'intérieur d'un espace intérieur, dans lequel le dispositif contient une couche de commutation, où la couche de commutation comprend un ou plusieurs composé(s) de la formule (I).

13. Dispositif selon la revendication 12, **caractérisé en ce que**, en plus du composé de la formule (I), un milieu cristallin liquide qui comprend un ou plusieurs composé(s) différents est présent dans la couche de commutation.

14. Dispositif selon la revendication 12 ou 13, **caractérisé en ce qu'**il peut être commuté électriquement.

15. Dispositif selon une ou plusieurs des revendications 12 à 14, **caractérisé en ce qu'**il est connecté à une cellule solaire ou à un autre dispositif pour la conversion de l'énergie lumineuse et/ou thermique en énergie électrique.
